# EUROPEAN PATENT APPLICATION

(11) **EP 2 074 999 A1**
(43) Date of publication of application: **01.07.2009**
(21) Application number: 07702271.3
(22) Date of filing: 05.02.2007
(51) Int. Cl.: A61K 31/52, A61K 31/70, A61K 38/19, A61K 31/202, A61P 35/02, A61P 35/00

(54) **USE OF RUBESCENSINE A AND DERIVATIVES THEREOF IN PHARMACY**

(30) Priority: 18.08.2006 CN 200610030200
(71) Applicant: Rui Jin Hospital Affiliated To Shanghai Jiao Tong University School of Medicine, Shanghai 200025 (CN)
(72) Inventor: CHEN, Zhu, Shanghai 200025 (CN); CHEN, Saijuan, Shanghai 200025 (CN); WANG, Zhenyi, Shanghai 200025 (CN); ZHOU, Guangbiao, Shanghai 200025 (CN)
(74) Representative: Schweiger, Martin
(86) International application number: PCT/CN2007/000380
(87) International publication number: WO 2008/022505

(57) **Abstract**

Use of Rubescensine A and derivatives thereof in pharmacy. Rubescensine A can induce the apoptosis of leukemia cells in vitro, and can prolong the survival period of mouse model of t(8;21) leukemia in vivo or delay the growth of tumor in body. Sequential, joint application of Rubescensine A and chemotherapeutics, such as cytarabine or agents for inducing differentiation may be more effective in prolonging the survival period of mouse of t(8;21) leukemia.

## Description

### TECHNICAL FIELD OF THE INVENTION

The present invention relates to the application of Rubescensine A, and more particularly to the application of Rubescensine A and its derivatives in the pharmaceutical field.

### BACKGROUND OF THE INVENTION

Myeloid leukemia includes acute myeloid leukemia (AML) and chronic myeloid leukemia (CML). Acute myeloid leukemia (AML), which is a heterogeneous hematological malignancy, is also the most common type of acute leukemia occurring in adults. According to records, in 2003 alone, approximately 11,000 Americans were diagnosed as having AML, of which approximately 75% of the patients eventually died as a result of the disease. In China, the number of newly diagnosed patients with acute leukemia is 40,000 cases annually. AML patients generally show blockage in normal differentiation and apoptosis of hematopoietic cells as well as abnormal proliferation of bone marrow hematopoietic stem/progenitor cells, leading to accumulation of immature progenitor cells in bone marrow and peripheral blood, affecting the formation and function of normal hematopoietic cells. As a result, patients may bleed, become anemic, and be prone to infection; furthermore, leukemia cells may infiltrate and disseminate in liver, spleen and other organs, ultimately leading to failure of patients' organs at which time death may occur.

In the past two decades, people have extensively studied AML in biology, molecular biology and cytogenetics, and such awareness has promoted great progress in the field, and in particular, discovery of chromosomal translocation involved in the occurrences of leukemia. For example, in 1982, M2-type AML patients were discovered to have specific t(8;21) translocation, and some 10 years later, the breaking point and the involvement of genes were discovered. Another type of leukemia, the acute promyelocytic leukemia (APL) which is the M3 type of FAB classification, is also a commonly seen blood cancer, which accounts for 10-15% of acute myeloid leukemia (AML) cases, wherein clinical manifestations of granulocytic differentiation is blocked at promyelocytic stage. In 1984, R. A. Larson and D. E. Hogge discovered the specific type of leukemia containing t(15;17) chromosomal translocation, and suggested that the translocation and bone marrow cell differentiation blocked in leukemia stage were related. In 1990-1991, several research teams reported nearly at the same time that t(15;17) generated the PML-RARα fusion gene, and then proved its potential to induce leukemia. At the same time, the treatments of AML have also made remarkable progress. For example, the traditional treatments for APL utilizes cytotoxic chemotherapy, which often leads to deadly intracranial hemorrhage in 8-46% of the patients. Until 1988, a discovery of an all-*trans* retinoic acid (ATRA) differentiation therapy of the disease had made a major breakthrough. The Shanghai Institute of Hematology firstly reported that ATRA alone can make t(15;17)-bearing APL patients achieve a complete remission. Subsequent studies on molecular mechanism discovered that ATRA can be partly combined with RARα of PML-RARα, which enables dissociation of transcription repressor, thereby restoring the RARα transcriptional function, allowing downstream target genes to be transcribed, so that the cells re-enter the differentiation process. Raelson et al. have discovered that ATRA treatment can degrade the specific PML-RARα fusion protein, so that the dominant negative effect of PML-RARα on the wild-type PML and RARα could be removed. During 1996-1997, the Chinese scholars demonstrated that arsenic trioxide (As₂O₃) not only can induce apoptosis of APL line NB4 cells, but also can significantly degrade PML-RARα fusion protein. More interestingly, low doses of As₂O₃ can partially induce differentiation of APL cell, while high doses can induce apoptosis. Clinical experiments have shown that both newly diagnosed and relapsed APL patients treated with As₂O₃ can achieve complete remission. In addition, ATRA jointly used with As₂O₃ not only shows an achievement of better results in APL mouse model, but also provides clinical treatment of early APL patients with better therapeutic effect than treating independently with ATRA or As₂O₃. ATRA and As₂O₃ as typical targeted therapies are being widely accepted. Currently, ATRA in combination with anthracycline compounds or arsenic trioxide allows the APL-specific t(15;17) chromosomal translocation of the M3-type AML to be cured in most patients. Although the application of cytosine arabinoside (Ara-CH, cytarabine) and anthracycline-containing chemotherapy, as well as advanced supportive care, makes other types of AML prognosis gradually improving that about 75-80% of AML patients receiving clinical remission, most patients will still have relapses. Stem cell transplantation is too expensive and with only limited donors, and transplant-related deaths can reach 25%-30%; therefore it was difficult to promote. Moreover, for older patients with AML, the effect of high dose chemotherapy is still unsatisfactory. Therefore, it is necessary to develop new drugs to treat such diseases. Apoptosis is an important mechanism of multi-cellular animals to remove harmful cells, and plays an important role in development, maturity of immune system, dynamic balance of organs and aging process, and thus developing drugs to effect selective induction of apoptosis of leukemia cells is a feasible pathway of treatment of AML.

Progress of Rubescensine A and Isodon diterpenoids in anti-tumor research: Isodon plants are widely used by the Chinese people as one of Chinese civil herbs, mostly for heat-clearing and detoxifying, activating blood stasis, anti-bacterial or anti-inflammatory, and anti-tumor efficacy. It is particularly noted that rubescens produced in Henan province, China, besides being used in anti-bacterial and anti-inflammatory applications, is also applicable to cancers, such as esophageal cancer and gastrio-cardial cancer. Such plants are rich in diterpene compounds, especially the uniquely structured mapping-kaurane-type diterpenoids. Rubescensine A (also called Oridonin) is an ent-kau-rene diterpenoid natural organic compound separated from the labiatae plant Isodon, and the compound contains α-methylene cyclopentanone-based structure that is related to anti-tumor activity. It has been reported that the molecule contains 7β-OH and 14β-OH, which are also considered having the effect of enhancing anti-tumor activity due to binding sites with special enzyme in tumor cells. Rubescensine A is a colorless prism-shaped crystal and does not dissolve in water, but it is soluble in organic solvents, including ether, methanol, and ethanol. People long ago had minimal awareness of Rubescensine A having a wide range of biological activity, such as immune modulation, antiviral, anti-inflammatory, and anti-tumor activity. Recent laboratory and experimental study of pre-clinical data suggests that Rubescensine A might have anti-tumor activity and might be effective against prostate cancer, breast cancer, non-small cell lung cancer and liver cancer, esophageal cancer, and pancreas cancer.

Overview of pathogenetic mechanism of M2-type leukemia: AML is a malignant blood disease resulting from proliferation of transformed bone marrow stem and progenitor cells, and chromosomal translocation is the most commonly seen cause leading to malignant transformation of hematopoietic progenitor cells, wherein t(8;21)(q22;q22) translocation accounts for about 10-20% of all acute leukemia cases, and t(8;21) is detected in 40%-80% of AML M2. Some FAB M4 type leukemia also contains t(8;21). t(8;21) translocation involves the genes AML1 and ETO, which forms AML1-ETO fusion gene due to translocation, and play a very important role in leukemogenesis of AML with t(8;21). Numerous studies discover that AML1 is also a target gene for other types of human leukemic chromosomal rearrangement, such as t(1;21), t(16;21), and t(12;21), forming fusion gene AML1-EVI-1, AML1-MTG16 and TEL-AML1, respectively. Through forming CBFβ-MYH11 fusion gene, inv(16) can destroy CBFβ, which is the partner of AML1 in forming a heterodimer, and indirectly affects the function of AML1. It is clearly evident that AML1 plays a very important role in the hematopoietic process. AML1 gene is located on the long arm of chromosome 21, zone 22. In normal circumstances, AML1 is expressed in hematopoietic tissue and myeloid differentiation, and also expressed in nervous tissues, skeletal muscles and regeneration of tissues. There are three known isomers of AML1 protein, namely AML1a (250aa), AML1b (453aa), and AML1c (480aa) (also called AML1B). AML1B has the following important domains. (1) Runt homology DNA binding domain (RHD), which is formed from about 118 amino acids of AML1 and is named because of its homology to Runt protein of Drosophila. Its combination with DNA has consensus sequence of TGT/cGGT. In addition, AML1 can also joint CBFβ through this domain to form heterodimer, which together regulate gene expression. (2) PST zone contains potential phosphorylated sites. (3) Nuclear Matrix Attachment Sequence (NM) may be related to the nuclear localization of AML1. (4) Two Transcriptional Activation Domains (TA), wherein the stronger functional one is located in the C-side of AML1, and the weaker functional one comprises PST and NM. (5) There are two possible transcription Repression Domains (RDs), wherein one is close to the C-terminal of RHD, and the other one is located between NM and TA. In addition, a conserved sequence VWRPY formed of five amino acids is located at the C-terminal of AML1, and can be combined with the transcription inhibitor Groucho/TLE1-4 to inhibit target gene transcription. AML1 works by forming heterodimer with CBFβ. CBFβ does not have the ability to combine with DNA, but can stabilize AML1 in combination with DNA. There are many hematopoiesis-related genes regulated by AML1, such as GM-CSFR, M-CSFR, IL-3, MPO, TCRβ, NE, and NP-3. Studies in recent years have discovered that only AML1 binding site is not enough to cause activity of gene promoter, and for the promoters of many genes, adjacent to AML1 binding site are often other transcription factors binding sites, such as Myb, Ets, AP1 and C/EBP. Therefore, it is believed that AML1/CBFβ functions as a transcription organizer. Through binding some gene enhancer, AML1/CBFβ can raise other transcription factors to have a formation with activated complex, such as being through P300/CBP coactivator histone acetyltransferase activity so to have chromatin histone acetylation, resulting in a loose chromatin structure to facilitate transcription. AML1 effect in the central regulation of hematopoiesis has been confirmed by AML1 knockout mice. AML1-/- mice are embryonic lethal, wherein first of all, death occurs in nerve tissue hemorrhage, followed by hematopoietic defects. AML1+/- mice show normal morphology, and comprises the original source of yolk sac erythrocytes. However, there is an absence of permanent hematopoietic progenitor cells in yolk sac and fetal liver. AML1 heterozygous myeloid in animals and erythroid progenitor cells are reduced, and the other allele inactivation in AML accounts for 2%. There have been two cases where two patients with two AML1 alleles simultaneously mutated at the same time, resulting in M0 type AML.

ETO function: ETO (Eight-Twenty-One) gene is located on the long arm of chromosome 8, zone 22, which encodes a 604 amino acid nuclear phosphoprotein. It is mainly expressed in the brain and CD34+ hematopoietic cells, and its specific functions are not very clear, and it is just discovered from the sequence analysis that it is homologous to Drosophila Nervy gene. Currently, there are four known important domains of ETO: (1) Homologous to Drosophila TAF110 transcription factor domain; (2) Hydrophobic Heptad Repeat (HHR); (3) Homologous Nervy domain; (4) Two zinc finger domains of C-side, wherein the latter one zinc finger is also known as MYNY domain. The above four domains are also known as NHR1-4, of which NHR2 can form amphiphilic helix, mediating homologous or heterologous dimerization, and this formation combination is quite stable. The two zinc finger domains of NHR4 can function with SMRT and N-CoR. The section comprising NHR2 and two sides (236-432 aa) thereof is known as CRD (Core Repressor Domain), which has a very strong effect with mSin3A. Currently, there is no evidence showing that ETO can combine with DNA, but it has been confirmed that ETO, through combination with inhibitors, such as N-CoR, SMRT, and mSin3A, can raise histone deacetylase (HDAC), enabling deacetylation of histone and making chromosomal structure closer, thereby inhibiting gene transcription.

The possible mechanism of AML1-ETO fusion gene leading to leukemia: t(8;21) generated ectopic AML1-ETO fusion protein, wherein AML1 N-terminal part (177 aa) and almost full-length ETO (C terminal 575 aa), thus can combine with CBFβ and form heterodimer, and also bind with DNA, and moreover, AML1-ETO and CBFβ affinity is greater than with the wild-type. Accordingly, the hypothesis of AML1-ETO fusion protein being a dominant inhibitor of AML1 is proposed, and experiments have proven that AML1-ETO inhibits the activation of the TCR β enhancer, IL3, and GM-CSF promoter induced by AML-1, but does not affect the background level expression of promoter. In addition, AML1-ETO heterozygous mice are embryonic lethal, almost identical with AML1 knockout mice phenotype, showing central and peripheral nerve hemorrhage and obstructed fetal liver hematopoiesis. There are several hypotheses regarding AML1-ETO transcriptional repression mechanism, for example, DNA binding site competition, effects on other surrounding factors, effects on inhibitors, or effects on end transcription machinery. Experiments of partial deletion of AML1-ETO show that ETO C-terminal sequence (comprising the HHR and MYND domain) is necessary for its inhibitory function. Moreover, any one single point mutation of the two zinc finger structures of the MYND domain will cause the loss of its inhibitory function, which suggests that AML1-ETO can mediate transcriptional inhibition through acting with other factors. As above mentioned, part of ETO can combine with inhibitors, such as N-CoR, SMRT, and mSin3A, forming one high molecular weight inhibitory complex, and then using the activity of its histone deacetylase activity to induce deacetylation of chromatin histone and makes the structure of chromatin closer, so that the transcription of some genes closely related to proliferation and differentiation of hematopoietic cells and can be inhibited, ultimately blocking differentiation of hematopoietic cell and causing abnormal proliferation, leading to leukemia. HDAC inhibitors weakening the AML1-ETO-mediated transcriptional repression may be supportive of this view. Other studies have discovered that AML1-ETO and AML1 can be synergetic to activate M-CSFR. The AML1-ETO-harboring Kasumi-1 cells show a higher expression of M-CSFR than monocytic cell line U937, while bone marrow from patients with M2-type has higher levels of M-CSFR than normal bone marrow. M-CSFR signal transduction through the induction of G1/S transition can promote cell proliferation. AML1-ETO can also activate Bcl-2 promoter, and so it may also possibly cause leukemia through promotion of cell proliferation and inhibition of apoptosis.

However, in AML1-ETO expression inducible transgenic mice, AML1-ETO expression does not cause leukemia; and in hematopoietic reconstitution experiments, the mice that have been irradiated by lethal dose and injected with AML1-ETO-expressing hematopoietic stem/progenitor cells are not capable of causing leukemia. These results suggest that the expression of AML1-ETO alone cannot cause leukemia, and it must be in collaboration with other gene mutations to cause AML to happen. Some experiments confirm the speculations. In AML1-ETO transgenic mice, the application of chemical mutagen ENU (N-ethyl-N-nitrosourea) can induce AML. There are evidences showing that t(8;21) leukemia is a gradual occurrence, with the generation of AML1-ETO fusion protein being the first step, which is an essential genetic change, while the C-KIT pathway activation being the second step, which is a crucial step in development of full-blown leukemia. In the afore-said model, the former step hinders the normal cell differentiation pathway, while the latter step grants the proliferation and/or anti-apoptotic capacity of hematopoietic cell; the synergy between the two steps may be the cause of leukemia.

Although currently there are a lot of studies about Rubescensine A, so far, there are no research reports on the application of Rubescensine A being used in manufacturing drugs for treating acute leukemia.

China patent application No. 2004100168916 discloses a method for degrading AML1-ETO fusion protein and reagents used thereof. The document only provides a general description of applicability of Rubescensine A in degrading AML1-ETO fusion protein. No documentation shows Rubescensine A for animal models or clinical trial data.

### SUMMARY OF THE INVENTION

The objectives of the present invention are: (1) providing an application of Rubescensine A in manufacturing drugs for treating acute leukemia; (2) providing an application of Rubescensine A in manufacturing drugs for treating chronic myelogenous leukemia; (3) application of Rubescensine A and cytarabine (cytosine arabinoside) jointly in manufacturing drugs for treating M2-type acute myeloid leukemia; (4) application of Rubescensine A derivatives in manufacturing drugs for treating acute leukemia; and (5) application of Rubescensine A in manufacturing drugs for treating other cancer.

The objectives of the present invention are achieved by the following methods: Studies on the effect of Rubescensine A on t(8;21) leukemia cells including Kasumi-1 cell line, U937 cells with transfected AML1-ETO (referred to as U937-A/E cells), and primary cells from patients with t(8;21) AML, studies on the effect of Rubescensine A on other leukemia cells, and studies on in vivo efficacy of two t(8;21) leukemia mouse models.

The study on the effect of Rubescensine A on leukemia cells with t(8;21) chromosomal translocation is divided into the following sections:

First, studies on in vitro apoptosis of t(8;21) leukemia cells induced by Rubescensine A are carried out and it is discovered that Rubescensine A can induce in vitro t(8;21) leukemia cell apoptosis.

Then, studies of the molecular mechanisms of Rubescensine A induced t(8;21) leukemia cells apoptosis are carried out. On the basis of Rubescensine A inducing in vitro t(8;21) leukemia cells apoptosis, it is attempted to discover how Rubescensine A induces in vitro t(8;21) leukemia cell apoptosis. The results show that Rubescensine A can induce the collapse of mitochondrial transmembrane potential of Kasumi-1 cells and the release of cytochrome C from mitochondria, and activates caspase-3 and caspase-9, showing that in the course that Rubescensine A induces apoptosis of Kasumi-1 cells, there exists endogenous apoptosis pathway activation. It is also found that in the course of apoptosis, there exists degradation of AML1-ETO fusion protein.

Then, studies on caspase-3 cleavage site on the AML1-ETO fusion protein are carried out. In the process of studying Rubescensine A inducing apoptosis of Kasumi-1 cells and other AML1-ETO fusion protein-positive cells (U937-A/E), it is discovered a phenomenon of AML1-ETO fusion protein degradation, and the AML1-ETO fusion protein plays a key role in the pathogenesis of leukemia. In the study of AML1-ETO fusion protein degradation mechanism, it is discovered that AML1-ETO fusion protein degradation is realized through incision with activated Caspase-3, and this is the first time to identify an important Caspase-3 incision site of Asp188.

Finally, the therapeutic efficacy of Rubescensine A on t(8;21) leukemia in mice and its mechanism are studied. As a truncated AML1-ETO (AEtr) transplantation mouse model and a Kasumi-1 cell subcutaneously transplanted tumor in nude mice are set up, these two models have demonstrated Rubescensine A having a therapeutic role. This confirms that Rubescensine A can prolong survival period of AEtr transplantation mice and can induce in vivo incidences of apoptosis of mouse spleen cells. Rubescensine A is also compared with cytarabine for therapeutic efficacy in AEtr transplant leukemia mouse model, and it is discovered that treatment with Rubescensine A at 2.5mg/kg/day dose for 10 days produces considerable therapeutic efficacy for AEtr transplantation mice as treatment with cytarabine at 25mg/kg/day for 5 days (low-dose cytarabine), and the does of 7.5 or 15 mg/kg/day Rubescensine A is more effective than low dose cytarabine. Although some leukemia mice (4/9) show better therapeutic efficacy with 25 mg/kg/day cytarabine for 10 days (high-dose cytarabine) in comparison to the efficacy of Rubescensine A, the other mice (5/9) have an early death (survival period of only 18 days). In contrast to a high-dose cytarabine, Rubescensine A at 2.5-15 mg/kg/day dose does not cause bone marrow suppression or weight loss in mice. Thus, Rubescensine A in the treatment of leukemia shows the features of having effective therapeutic efficacy and less drug side effects. In the nude mouse Kasumi-1 cell subcutaneously transplanted tumor model, Rubescensine A can significantly inhibit the growth of subcutaneous transplanted tumor, which also shows the in vivo anti-leukemia effect of Rubescensine A.

In addition, via in vitro and in vivo identification of the anti-t(8;21) leukemia effect of Rubescensine A, further studies of improving the therapeutic efficacy of Rubescensine A are carried out, and based on the principle of multiple target induced leukemia cells in molecular target therapy, joint administration of drugs is studied and synergy between drugs is explored in order to improve the anti-leukemia effect of Rubescensine A. Rubescensine A is used in combination with low doses of traditional chemotherapy drug, cytarabine (25mg/kg/day IP), in therapeutic treatment of AEtr leukemia mice, and in comparison with treatment solely with Rubescensine A, the survival period is significantly prolonged. With differentiation inducing drug [Granulocyte colony-stimulating factor G-CSF (50mg/kg/day SC) and all-trans retinoic acid (ATRA) (10mg/kg/day IP)] sequentially and jointly applied in treatment of leukemia mice, compared with groups of Rubescensine A (7.5mg/kg/day IP) administration alone and control groups that receive no drug administration, survival period is significantly prolonged, which demonstrates a clear anti-t(8;21) leukemia effect.

The above studies show:

(1) The application of Rubescensine A in manufacturing drugs for treating acute leukemia.

(2) The application of Rubescensine A in manufacturing drugs for treating acute myeloid leukemia with AML1-ETO fusion gene.

(3) The application of Rubescensine A in manufacturing drugs for treating acute myeloid leukemia not containing AML1-ETO fusion gene.

(4) The application of Rubescensine A in manufacturing drugs for treating acute monocytic leukemia.

(5) The application of Rubescensine A in manufacturing drugs for treating acute promyelocytic leukemia.

(6) The application of Rubescensine A in manufacturing drugs for treating chronic myelogenous leukemia.

(7) Rubescensine A treatment does not cause bone marrow suppression or weight loss, and is a relatively safe anti-leukemia drug.

(8) Sequential and joint application of Rubescensine A with chemotherapy drug cytarabine and/or differentiation-inducing drug (granulocyte colony-stimulating factor (G-CSF) and all-trans retinoic acid (ATRA)) can have synergetic anti-leukemia effect. It is an effective therapeutic solution for improving the efficacy of Rubescensine A in the treatment of t(8;21) leukemia.

Rubescensine A derivatives can extend the survival period of AEtr transplant mice, inhibit the growth of Kasumi-1 cells subcutaneously transplanted tumor in nude mice, and also can significantly reduce leukemia cell infiltration in liver, spleen and bone marrow in these two mouse models, suggesting that Rubescensine A derivatives may have a very strong anti-leukemia effect. For the application of Rubescensine A derivatives in manufacturing drugs for treating acute leukemia, the Rubescensine A derivatives used are: Rubescensine A Schiff bases, amino acids, sugars, nucleosides, fluorine, and derivatives produced by structure simplification, but the Rubescensine A derivatives used are not limited to the above-mentioned derivatives.

Abnormality of endogenous apoptosis pathways causing blocked apoptosis is the basic characteristics of tumors, and Rubescensine A can activate key protein caspase-3 in apoptosis pathway, so that the cytochrome C can be released from mitochondria, causing reduction of mitochondrial transmembrane potential, and thus Rubescensine A can be applied to the treatment of cancers other than leukemic, including treating the esophagus cancer, stomach cancer, colon cancer, liver cancer, lung cancer, pancreatic cancer, prostate cancer, melanoma, and nasopharyngeal carcinoma.

The present invention discloses applications of Rubescensine A and Rubescensine A derivatives in manufacturing drugs, having the advantages of:

(1) The present invention discovers new medical purposes for Rubescensine A and opens a new application area.

(2) Rubescensine A of the present invention is safe and non-toxic, and has strong pharmacological effects, indicating a very good prospect of medicinal future.

(3) Rubescensine A can induce in vitro t(8;21) leukemia cell apoptosis.

(4) The process of Rubescensine A inducing kasumi-1 cell apoptosis involves activation of endogenous apoptosis pathway. Also, in the course of apoptosis, there exists degradation of AML1-ETO fusion protein.

(5) It is discovered that the degradation of AML1-ETO fusion protein by Rubescensine A is realized through cleavage carried out by activated caspase-3, and it is for the first time to identify an important caspase-3 incision site Asp188.

(6) AEtr transplant mouse model and nude mouse Kasumi-1 cell subcutaneously transplanted tumor model are established. Leukemia cells separated from spleen of the AEtr mice can be induced to undergo apoptosis by adding Rubescensine A when cultured in vitro. Further, in vivo studies show Rubescensine A can extend the survival period of AEtr transplant mice, inhibit the growth of Kasumi-1 cell subcutaneously transplanted tumor in nude mice, and further, in these two mouse models, infiltration of leukemia cells in liver, spleen and bone marrow can be significantly reduced, suggesting that Rubescensine A has a strong anti-leukemia effect.

(7) In vivo study of sequential and joint application of Rubescensine A with chemotherapy drug cytarabine and/or differentiation inducing drug (granulocyte colony-stimulating factor, G-CSF, (50mg/kg/day SC) and the all-trans retinoic acid, ATRA, (10mg/kg/day IP)) in the AEtr transplant mouse model set up above shows that combined application of the above discussed drugs and Rubescensine A can significantly prolong the survival period of the AEtr transplanted mice and reduce infiltration of leukemia cells in liver, spleen and bone marrow of the mice. Excellent synergy in anti-leukemia is demonstrated.

(8) Rubescensine A can cause reduction of mitochondrial transmembrane potential, release of cytochrome C from mitochondria, and activate key protein caspase-3, and thus Rubescensine A can be applied to the treatment of cancers other than leukemia.

### BRIEF DESCRIPTION OF THE DRAWINGS

FIG.1 shows chemical structure of Rubescensine A (C₂₀H₂₈O₆);

FIG. 2A shows Rubescensine A inducing t(8;21) leukemia cell apoptosis, wherein Kasumi-1 cells are treated with Rubescensine A and then stained with Wright staining dye and are thereafter observed under a microscope for morphology examination, which indicates characteristic of apoptosis;

FIG. 2B shows apoptosis analyzed by flow cytometry after Annexin V/PI double-staining;

FIG. 2C shows apoptotic proportion of Kasumi-1 cells treated with Rubescensine A (Annexin V-positive ratio);

FIG. 3 shows Rubescensine A significantly decreasing Kasumi-1 cells PI (-)Rh123-positive cells, indicating it can induce transmembrane potential precursors to collapse, this effect being time- and dose-dependent;

FIG. 4 shows Western Blot results indicating that after Rubescensine A treatment of Kasumi-1 cells for 4 hours, release of cytochrome C and activation of Casapse-3 and Casapse-9 are found and degradation of Casapse-3 substrate PARP is seen, these changes being time-dependent with the most significant changes detected in 24 hours;

FIG. 5 shows (A) Western Blot detection of Rubescensine A caused degradation of AML1-ETO fusion protein in Kasumi-1 cells; (B) Western Blot detection showing Rubescensine A can induce degradation of AML1-ETO protein of U937 cell line expressing AML1-ETO fusion protein (U937-A/E);

FIG. 6 shows Caspase-3 specific inhibitor Z-DQMD-FMK prevents degradation of AML1-ETO fusion protein caused by Rubescensine A, arrow indicating the degradation fragments;

FIG. 7 shows no AML1-ETO cleavage fragments is detected in U937 cells expressing Asp188Ala mutations in AML1-ETO, while mutation in Asp171 or Asp192 can not abrogate the degradation.

FIG. 8 shows that Rubescensine A soluibized in 1% F68 (pluronic) (ori/P) demonstrates a similar inhibition rate on Kasumi-1 cells as Rubescensine A dissolved in DMSO (ori);

FIG. 9 shows pathological examination of nude mice inoculated with Kasumi-1 cell subcutaneously.

FIG. 10 shows pathological inspection of spleen, liver, and bone marrow of Kasumi-1 cells-harboring nude mice treated with/without Rubescensine A. The results show that Rubescensine A significantly reduces infiltration and propagation of leukemia cells and prevents structure damage of spleen, liver, and bone marrow.

FIG. 11 shows Rubescensine A can inhibit the growth of Kasumi-1 cells in nude mice;

FIG. 12A shows that, compared to normal C57 mice, peripheral blood and bone marrow of mice injected with AEtr-expressing cells are filled with a large number of immature cells;

FIG. 12B shows liver and spleen of mice bearing AEtr-expressing cells are infiltrated with a large number of abnormal cells;

FIG. 13 shows that with in vitro treatment of Rubescensine A for 24 hours, leukemia cells separated from spleen of AEtr transplant mice appear a large number of apoptotic cells;

FIG. 14A shows biopsy suggesting that after treatment with Rubescensine A, leukemia cell infiltration is significantly inhibited in liver and spleen of AEtr transplant mice;

FIG. 14B shows that, in comparison with the control group, after treatment with Rubescensine A, splenomegaly is significantly reduced in AEtr transplant mice;

FIG. 14C shows smear of peripheral blood indicating the control mice have a large number of leukemic cells, and Rubescensine A treatment significantly reduces the cells;

FIG. 15 shows Rubescensine A treatment significantly prolongs the survival period of AEtr transplanted mice. The control group is labeled "Control" (n=11), Rubescensine A 2.5 mg/kg/day group is labeled "Ori 2.5" (n=8), Rubescensine A 7.5 mg/kg/day group is labeled "Ori 7.5" (n=12, Rubescensine A 15 mg/kg/day group is labeled "Ori 15" (n=8), the high-dose cytarabine group is labeled "Ara-C-H" (n=9, and the low-dose cytarabine group labeled "Ara-C-L" (n=6).

FIG. 16 shows combined use of Rubescensine A and cytarabine can exert synergistic effect and significantly prolong the survival period of mice harboring AEtr-expressing cells.

FIG. 17 shows combined use of Rubescensine A and cytarabine in treating mice harboring AEtr-expressing cells can reduce leukemia cell infiltration in the peripheral blood, liver, and spleen

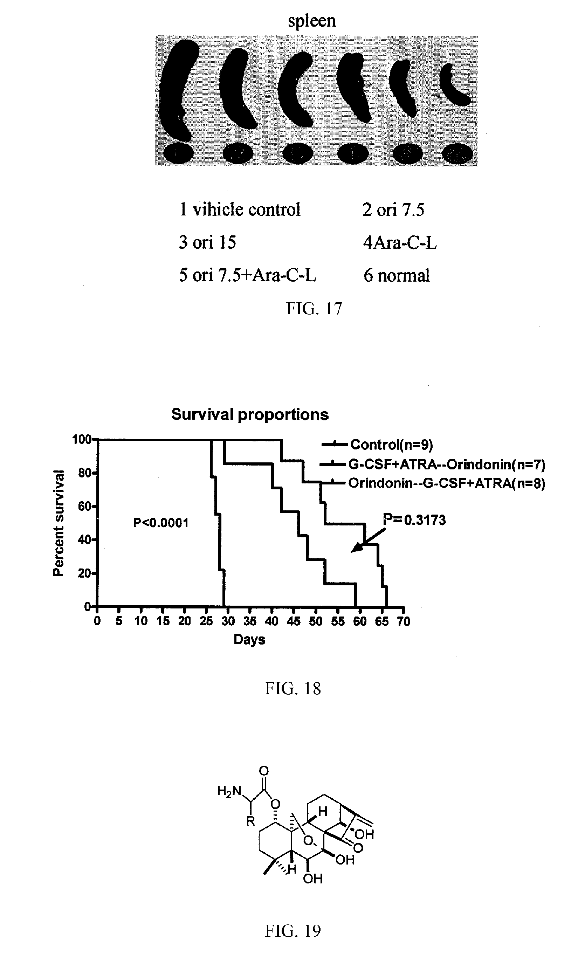

FIG. 18 shows sequential and simultaneously application of Rubescensine A (7.5mg/kg/day, IP) and G-CSF (50mg/kg/day, SC)+ATRA (10mg/kg/day, IP) can significantly prolong the survival period of AEtr transplantation mice. Treatment protocols: control group (n=9): treated with vehicle control only; G-CSF+ATRA→ori group: treatment with G-CSF (50mg/kg/day, SC)+ATRA (10mg/kg/day IP) for 3 days then Rubescensine A 3 days then G-CSF (50mg/kg/day SC)+ATRA (10mg/kg/day IP) for 4 days followed by Rubescensine A 4 days (n=7); ori→G-CSF+ATRA group: Rubescensine A for 3 days, then G-CSF (50mg/kg/day)+ATRA (10mg/kg/day) for 3 days, then Rubescensine A 4 days and then G-CSF (50mg/kg/day)+ATRA (10mg/kg/day) for 4 days (n=8).

FIG. 19 shows chemical structure of Rubescensine A amino acid derivative;

FIG. 20 shows a schematic diagram (flow chart) of a method for synthesizing Rubescensine A amino derivative;

FIG. 21 shows chemical structure of Rubescensine A Schiff base derivative;

FIG. 22 shows a schematic diagram (flow chart) of a method for synthesizing Rubescensine A Schiff base derivative;

FIG. 23 shows chemical structure of Rubescensine A glycosyl derivative;

FIG. 24 shows a schematic diagram (flow chart) of a method for synthesizing Rubescensine A glycosyl derivative;

FIG. 25A shows chemical structure of Rubescensine A fluorine-containing derivative I;

FIG. 25B shows chemical structure of Rubescensine A fluorine-containing derivative II; and

FIG. 25C shows chemical structure of Rubescensine A fluorine-containing derivative III.

### DETAILED DESCRIPTION OF PREFERRED EMBODIMENTS

The present invention will be described below with reference to the embodiments thereof.

### Embodiment 1: In vitro study of Rubescensine A inducing apoptosis of t(8;21) leukemia cell

I. Materials and Methods:

1. Reagents and equipment: Rubescensine A with a purity of 98%-99.8% is subjected to dissolution by application of DMSO (Sigma) to prepare a stock solution of 10⁻²mol/L concentration and stored at -20°C. Propidium iodide (PI), which is a product of American company Sigma, is mixed with triple-distilled water to make 250 µg/mL stock solution stored at 4°C and avoiding light. Fetal bovine serum (FBS) is purchased from American company HyClone. Annexin V Test Kit (ApoAlert Annexin-V kit) is purchased from American company BD Biosciences. Flow cytometer is a product of American company Beckman Coulter. Fluorescence microscope (Olympus, BX60) and phase contrast microscope (Olympus, IMT) are purchased from Japanese company Olympus Corporation. Smear instrument cytospin is purchased from British company Shandon.

2. Cell culture and treatment and morphological observation of cells: Human t(8;21) leukemia cell line Kasumi-1 (purchased from United States Biological Resource Center, ATCC (the American Type Culture Collection)) are incubated, with an initial concentration of 2×10⁵/ml, in RPMI1640 cell culture medium containing 10% FBS and normally cultured under the conditions of 37°C, 5% CO₂, and saturated humidity. In the exponential growth period of Kasumi-1 cells, the cell density is adjusted to 3×10⁵/ml, and is applied with treatment of Rubescensine A, in which specific application of three concentrations, 0.5µM, 2µM, and 5µM, is given and in 5µm concentration, time pints of 0h, 4h, 8h, 12h, and 24h are selected. The control group is added with DMSO of which the concentration is the maximum DMSO concentration of the Rubescensine A treatment groups. After the treatment is completed, smear is performed with Cytopro centrifuge and then dried, followed by Wright staining to thereafter carry out cell morphology with a microscope.

3. Apoptosis inspection with Annexin V method: Operations are carried out in accordance with the Annexin-V Test Kit specification, with the following steps:

(1) Harvesting 10×10⁵ Kasumi-1 cells, where the cells are washed two times with pre-cooled 1×PBS and afterwards washed one time with Binding Buffer from the Test Kit and re-suspended in 100µL Binding Buffer;

(2) Adding 5µL Annexin V FITC reagent and 5µL PI, gently mixing, and incubating at room temperature for 15-20 minutes in a condition of avoiding light; and

(3) Adding 400µL Binding Buffer, and carrying out inspection with flow cytometer in one hour.

II. Results and discussion:

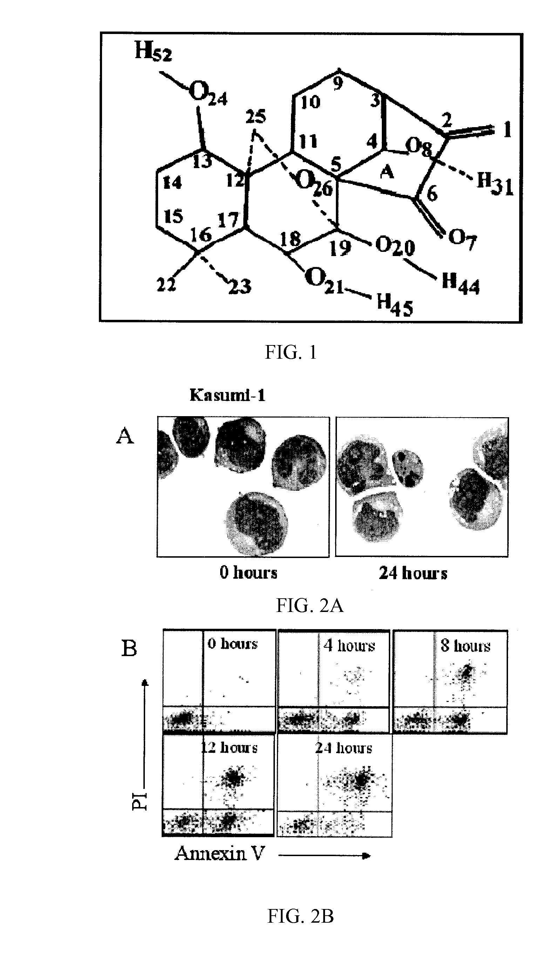

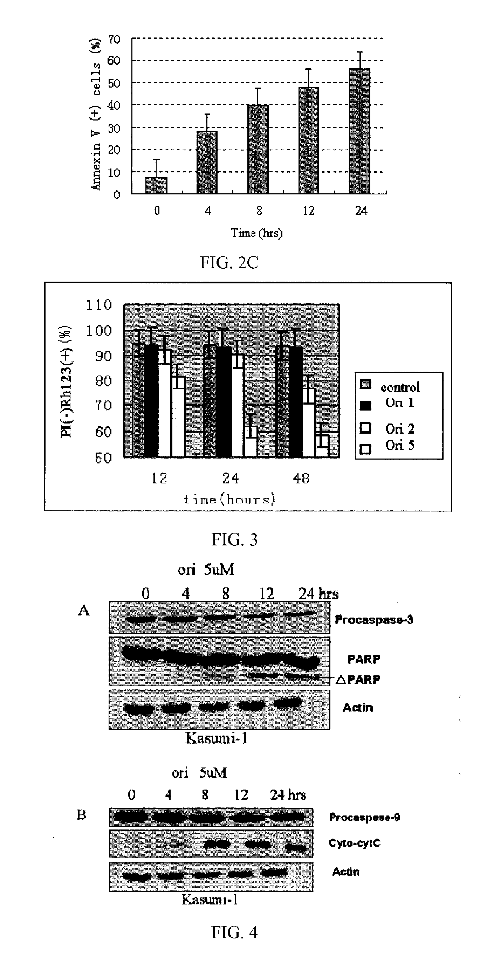

Rubescensine A can induce apoptosis of Kasumi-1 cell, and this process is dose-and time-dependent. The Rubescensine A treated Kasumi-1 cells show morphologically significant apoptotic features, such as chromatin condensation, nuclear breakdown and cell membrane maintaining integrity. In the apoptotic process, the imbalance distribution of phospholipids in bimolecular layer of cell membrane is destroyed, and eversion of phosphatidylserine (PS) from the inner layer to the outer layer occurs, whereby the phosphatidylserine exposed on the outer surface may be recognized and engulfed by the nearby phagocyte, so that apoptotic cells can be cleared and removed. Annexin V is a phospholipid-binding protein, which can specifically identify the everted phosphatidylserine on the cell surface, and so it is often used for detection of apoptosis. PI is a red fluorescent dye which can combine DNA, but only in cells undergoing necrosis, under cell membrane rupture circumstances, it can go through the cell membrane and bind with DNA. Therefore, flow cytometry analysis for double-staining of Annexin V-FITC/PI is the most sensitive and specific method for detecting apoptosis of suspending cells. Double-labeled Annexin V-FITC/PI shows that Annexin V(+)/PI(-) cells and the effect of Rubescensine A are closely related, showing time-dependency. After treatment for 4 hours, Annexin V(+)/PI(-) cells appear (Figure 2B), and this type of cells will increase its number with extension of time.

In 1972, J. F. Kerr was first to propose the concept of apoptosis, which, in ancient Greek, means a process of death like autumn leaves withering. He discovered that ligation of the left and middle portal vein of rat liver causes avascular necrosis nearby cells, but the hepatic artery supply areas parenchymal cells are still alive, only gradually narrowing the scope, during which cells successively change into small pieces of cytoplasm without occurrences of inflammation, which was different from cell necrosis. In apoptosis, the morphology of early nuclear pyknosis, chromatins assembled in the nuclear membrane showing a shape of medial meniscus and nuclear fragmentation; cytoplasm and the cell density increased, cells sizes become smaller; normal or mild swelling of mitochondria; membranes folded and curled up, early membrane integrity was not damaged; membrane blebbing, budding, generating membrane coated apoptotic bodies, containing the complete organelle and nuclear debris, often rapidly phagocytosis by neighboring cells, surrounding tissue do not show inflammatory reaction. Subsequent studies discovered that apoptosis is a widespread phenomenon, and it is a natural mechanism of multi-cellular organisms to remove the body's non-useful and damaged cells, for example, during amphibian embryonic development the disappearance of gills and tail and non-lactating mammary gland degradation process which apoptosis will occur. In normal circumstances, the distribution of phospholipids in the cell membrane is uneven, phosphatidylcholine and sphingomyelin are mainly distributed in the outer membrane, and phosphatidylethanolamine and phosphate-dylserine are distributed in endometrium. Y. Tanaka, et al. and L. McEvoy, et al. discovered that damaged and aging red blood cells show PS eversion on the surface, which can be specifically recognized and engulfed by macrophage. In 1992, V. A. Fadok, et al. discovered that the asymmetrically distributed phospholipids of apoptotic lymphocytes are damaged, making them removed by macrophages. It was subsequently discovered that PS eversion was a common characteristic of apoptotic cells. Annexin V is a Ca₂+ ion-dependent phospholipid binding protein, and H. A. Andree, et al. discovered Annexin V will take priority to bind with negatively charged PS. G. Koopman, et al. for the first time in 1992 reported the method of using FITC-coupled Annexin V to mark the everted PS on the surface of apoptotic cell, followed by employing flow cytometry. They discovered that propidium iodide, which is a fluorescent dye which can go through damaged cell membrane to bind with DNA, marks only part of Annexin V-positive cells, which states that PS eversion was an early occurrence in apoptosis and thereafter, cell membrane rupture occurs. Now, Annexin V-FITC/PI double-standard method has become a standardized method to detect cell apoptosis. The present inventors' research results show that Rubescensine A, as a type of diterpenoids compound, can induce Kasumi-1 cells to enter the apoptosis process. Morphological observation reveals that Rubescensine A treated Kasumi-1 cells show apoptotic features of nuclear pyknosis, fragmentation but cell membrane being intact. Further, applying Annexin V/PI double-staining detection also confirms Rubescensine A can induce apoptosis in Kasumi-1 cells, and this process is time-dependent.

### Embodiment 2: Study of molecular mechanism of Rubescensine A inducing kasumi-1 apoptosis

I. Materials and Methods:

1. Reagents and equipment: For Rubescensine A, propidium iodide (PI), and fetal bovine serum, referring to "Materials and Methods" section of Embodiment 1. Rhodamine (Rh123) is a product of American company Sigma, and rhodamine is mixed with triple-distilled water to form 10µg/mL stock solution, stored at 4°C and avoiding light. Caspase-3 specific inhibitor Z-DQMD-FMK is a product of American company Sigma, and is pre-dissolved in DMSO and stored at -20°C before use. Adenosinediphosphate (ADP)-ribose poly-ADP-ribose-polymerase (PARP) antibody and anti-ETO antibody are purchased from American company Santa Cruz Biotech. Anti-Caspase-3 and Caspase-9 antibody, horseradish peroxide complex enzyme (HRP) labeled secondary antibody, and Western-blot color system are purchased from American company Cell Signaling. Actin antibodies are purchased from American company Sigma. Cytochrome C Releasing Apoptosis Assay Kit is a product of BioVision company. Nitrocellulose membrane is a product of British company Amarsham Biosciences. Ponasterone A is a product of American company Invitrogen. For flow cytometer and smear instrument, referring to "Materials and Methods" section of Embodiment 1.

2. Cell culture and treatment: Culture of Kasumi-1 cell is similar to Embodiment 1, and in the experiment, cells are inoculated and cultured at the density of 2-4×10⁵/ml. Rubescensine A of three concentrations, 1µM, 2µM, and 5µM, is applied. The control group is applied with the maximum volume of DMSO applied in the treatment groups to treat Kasumi-1 cells for 24-48 hours and thereafter, detection of mitochondrial transmembrane potential is performed. Rubescensine A at 5µm concentration is applied to treat Kasumi-1 cells and the cells are harvested at time points of 0, 4, 8, 12, and 24 hours. The harvested cells are subjected to total protein immunoblotting experiments. Rubescensine A of two concentrations, 2µM and 5µM, with/without 50µM Caspase-3 specific inhibitor Z-DQMD-FMK, is applied for incubation and observation of cell morphology. Culture of stably transfecting cell lines (U937-A/E) for inducing expression of AML1-ETO fusion protein is the same as Kasumi-1, and before treatment with Rubescensine A, treatment with Ponasterone A (PA) 5 ul/ml for 12 hours is applied to induce expression of AML1-ETO fusion protein. Cells are harvested at time points of 0, 4, 8, 12, 24, and 48 hours after treatment with Rubescensine A. The harvested cells are subjected to total protein immunoblotting experiments. Smear instrument is employed to collect cells on glass slides and then Wright staining dye is used and observation with optical microscope is carried out.

3. Flow cytometry analysis of mitochondrial transmembrane potential change: Detection of mitochondrial transmembrane potential uses Rh123 (10µg/mL) to detect mitochondrial transmembrane potential, with the following steps:

(1) Harvesting 1×10⁶-2×10⁶ kasumi-1 cells, which are washed two times with pre-cooled 1×PBS and then added with 100µL Rh123, mixed gently, and incubated for 30 minutes at 37°C in the condition of avoiding light;

(2) Washing two times with pre-cooling 1×PBS and then adding 5µL PI, gently mixing, reaction at room temperature for 15 minutes in the condition of avoiding light; and

(3) Adding 400µL 1×PBS and carrying out inspection with flow cytometer.

4. Analysis of cytochrome C release: Application of cytochrome C release apoptosis assay kit, with the following steps:

(1) Harvesting 5×10⁷ cells under the conditions of 600g, 5 minutes, and 4°C, washing one time with pre-cooled 1×PBS;

(2) Using DTT and protease inhibitors added 1×Cytosol Extraction Buffer Mix 1ml to re-suspend cells, and placed on ice for 10 minutes;

(3) Using pre-cooled tissue for homogenate broken cells, to ensure cell membrane broken but cell nuclear complete, approximately 30-50 times;

(4) Transferring homogenate to 1.5ml centrifuge tubes, 700g, centrifuge for 10 minutes at 4°C;

(5) Collecting supernatant in a new 1.5 ml centrifuge tube, 10000g, centrifuge for 30 minutes at 4°C;

(6) Collecting supernatant, which is non-mitochondrial cytoplasm components; using DTT and protease inhibitors added Mitochondrial Extraction Buffer Mix 0.1ml to re-suspend sediment, vibrating to uniformly mix mitochondrial components for 10 seconds; and

(7) Applying 10µg of non-mitochondrial cytoplasm component and mitochondrial component to proceed with routine Western blot experiment (choosing 12% polyacrylamide gel electrophoresis; applying anti-cytochrome C antibody provided in the Test Kit).

5. Western Blot (WB): The main steps are as follows:

(1) Harvesting cells, washing two times with pre-cooled 1 × PBS, adding 80µL protein lysis buffer (1×SDS protein sample buffer) to each 1×10⁶ cells, gently mixing, cooking 5-8 minutes in boiling water, obtaining sample or packing and cryogenically storing at -20°C;

(2) Polyacrylamide gel electrophoresis (SDS-PAGE) separation of proteins, wherein suitable concentration of gel (8%-12%) is selected in accordance with the target protein molecular weight , and normal electrophoresis conditions (80V voltage bromophenol blue to separation gel then switch to 120V) are employed;

(3) Applying wet-transfer method to transfer protein from the gel to 0.22µm nitrocellulose membrane;

(4) Using 5% skimmed milk powder-TBS to seal nitrocellulose membrane overnight;

(5) Incubation of antibodies: anti-I incubation, with selected polyclonal or monoclonal antibodies specific to incubate nitrocellulose membrane, at room temperature, for 2 hours, at 70 rpm vibration; with TBS-Tween (0.1 %) to wash membrane for 15 minutes, two times; anti-II incubation, select corresponding anti-nitrocellulose membrane to incubate, at room temperature, for 1.5 hours, at 70 rpm vibration; with TBS-Tween (0.1%) to wash membrane two times for 15 minutes;

(6) Color, applying a horseradish peroxidase substrate chromogenic system to color; and

(7) photo-sensing, developing, and fixing of X-ray films.

II. Results and discussion:

1. in Rubescensine A induced Kasumi-1 apoptosis process, it is accompanied by the collapse of mitochondrial transmembrane potential, and induction of Caspases-mediated endogenous apoptosis pathway.

In order to clarify the mechanism which Rubescensine A induces apoptosis of Kasumi-1, the researches first identify the effect of the compound on mitochondrial transmembrane potential (Δψm) through inspection with PI and Rh123 double-staining. Rh123 is a lipophilic cation, capable of emitting green fluorescence, and it can be absorbed by mitochondria. A normal living cell, due to high mitochondria Δψm, has a high intake of Rh123, and therefore, the cell will emit green fluorescence. When apoptosis occurs in a cell, mitochondrial Δψm becomes low, leading to a reduction of absorption of Rh123, whereby the cell shows low staining of Rh123. After Kasumi-1 cells are treated with 5um Rubescensine A for 12 hours (Figure 3), PI-negative but low staining Rh123 cells began to appear, and increase in number with the treatment time and dosage of Rubescensine A.

Apoptosis is cell death process that is subjected to strict control by related genes. Based on the sources of apoptosis signals, the apoptosis pathway can be divided into two pathways: exogenous pathway (death receptor pathway) and endogenous pathway (mitochondrial pathway). These two pathways will finally converge on the downstream effective Caspase. In the implementation phase of apoptosis, the effective Caspase can directly cause degradation of important proteins of cells and activation of nuclease, eventually leading to cell apoptosis. The present invention employs Westen Blot to detect the changes in apoptosis-related proteins, as shown in Figure 4. It can see that activation of Caspase-3 and the activation of the upstream Caspase-9 and degradation of important substrate PARP of Caspase-3 at the time points of 0, 4, 8, 12, and 24 hours after the application of 5µm concentration Rubescensine A to treat Kasumi-1 cells, and these changes show significant relevance to the concentration and time of the treatment of Rubescensine A. It is detected that cytochrome C is released within mitochondrial, and experiments show that after Rubescensine A is applied to Kasumi-1, cytochrome C of mitochondrial will be released to the cytoplasm.

2. Rubescensine A can cause degradation of AML1-ETO fusion protein in Kasumi-1 cells and in induced expression of AML1-ETO in U937 cells.

Rubescensine A can cause degradation of AML1-ETO fusion protein of Kasumi-1 cells, wherein the full-length size of AML1-ETO fusion protein is approximately 94KD and the size of AML1-ETO degradation fragment (ΔAML1-ETO, ΔAE) is about 70KD. In the U937 cell line (U937-A/E) that is applied with induced expression of AML1-ETO fusion protein, it can also be observed the effect of Rubescensine A on AML1-ETO fusion protein.

Apoptosis is a cell death process that is subjected to strict control by series of related genes. Different apoptotic signals may induce different apoptotic signal transduction pathways in cells. Based on the sources of apoptosis signals, apoptosis signal transduction pathways can be divided into two main pathways: (1) Exogenous pathway (death receptor pathway) and endogenous pathway (mitochondrial pathway). These two pathways will finally converge on the downstream effector molecules of Caspase. Effector Caspase in the implementation phase of apoptosis can directly give raise to degradation of important proteins and activation of nuclease activation, eventually leading to apoptosis. Exogenous apoptosis pathway mainly refers to a death receptor pathway. Under certain stimulation of receptor-induced signals, death receptors on cell membrane binds with the corresponding extracellular ligands, whereby the receptor binds with the cytoplasmic adapter protein and Caspase-8/-10 precursor protein to form oligomeric body, and such oligomers are referred to as "apoptotic bodies". Activating Caspase-8/-10 in the apoptotic body may further activate downstream effector Caspase (3, 6, 7), causing apoptosis. (2) Endogenous pathway (mitochondrial pathway). Un-repairable genome damage inside a cell caused by mutagenic agents, chemotherapy drugs and ionizing radiation may activate the endogenous pathway of apoptosis, in other words, the mitochondrial pathway. Various forms of stimulatory signals acting on mitochondria will cause mitochondrial to release pro-apoptotic factors, such as cytochrome C, apoptosis-inducing factor (AIF), and Caspase precursor protein (2, 3, 8, 9). Cytochrome C, after being released from mitochondria, forms a complex with Apaf-1 (apoptosis protease activating factor-1), and this complex attracts Caspase-9 precursor to combine into the complex, and thereafter, Caspase-9 is activated. The activated Caspase-9 in turn acts on the downstream effector Caspase (-3, 6, 7), causing cell apoptosis. However, these two pathways, the endogenous pathway and the exogenous pathway, do not exist in isolation, and there exists interaction therebetween. In addition to the above-mentioned two major apoptotic pathways, there are some other apoptosis pathways. Junying Yuan, et al. have discovered that endoplasmic reticulum is another organelle which plays an important role in the implementation process of apoptosis, and in certain cell types, the endoplasmic reticulum is an important member of the endogenous apoptosis pathway. For example, calcium-ion channels and Caspase-12 all participate in this process. Moreover, injuries of the nucleus can also lead to cell apoptosis. Mutagenic agents, chemotherapy drugs, and ionizing radiation may cause cellular DNA damage, leading to apoptosis. Cytoskeletal system is also an apoptosis receptor.

Caspases are a class of cysteine-specific aspartate protease (Caspase). In Caspase protease, a part of which has nothing to do with apoptosis, rather, is related to cytokine processing and matuation, including Caspase-1, -4, -5, -11, -12 and -13. Another part is involved in apoptosis, and this part of Caspase can be further divided into two categories, that is the initiators of Caspase and the effectors of Caspase. The initiators of Caspase are responsible for receiving apoptosis signal from the upstream, its activation process being the apoptosis initiation process, and include Caspase-2, -8, -9, -10. The effectors of Caspase are located downstream the apoptotic signal transduction pathway, responsible for implementing the apoptosis process, and include Caspase-3, -6, -7. Caspase can be activated by a number of molecules in the "death receptor" pathway. FAS/CD95 and TNFR1 apoptotic functions are directly dependent on Caspase. Caspase can also be activated by the mitochondrial pathway. Mitochondrial injury or other reasons leads to the release of mitochondrial apoptotic factors, including cytochrome C, Apaf-1, and AIF, and the likes. In the presence of Cytochrome C and ATP, Apaf-1 combines, through its N terminal CARD, with Caspase-9 corresponding homologous sequence, to activate Caspase-9 and thus induce apoptosis. Once the cells are in the event of apoptosis, often a series of Caspase are activated. Therefore, besides the above mentioned several forms of Caspase activation, Caspase molecules of the same type can also combine into a self-activated polymer, or rely on the other activated Caspase to become activated, thereby triggering a series of Caspase cascade activation, leading to cell apoptosis. The role of the activated Caspase generally includes three categories: One is to degrade those proteins that show protective effects on cells, thus de-activating apoptosis inhibitor protein. For example, Caspase can degrade apoptotic inhibitor protein Bcl-2/Bcl-xl, changing it into pro-apoptotic protein. The second channel is to destroy cell structure through direct degradation of structure protein apoptotic cell. For example, caspase can destruct cell structure through degrading nuclear lamina. Nuclear lamin protein forms lamin skeleton under the nuclear membrane to support the nuclear membrane and also affecting the chromatin structure and nuclear functions. In the occurrence of apoptosis, nuclear lamin is cleaved by Caspase, leading to typical variation of apoptosis, such as collapse of the nuclear skeleton and chromatin condensation. The third specific function of Caspase is to cleave certain important target proteins that have the features of maintaining cell homeostasis and nuclear repairing, such as nuclear DNA repairing proteins, DNA replication protein, mRNA sheared protein, and so on.

The experiment results show that after 24-48 hours when Rubescensine A acts on Kasumi-1 cells, Caspase-3 of the cells is activated, accompanied by PARP degradation of an important substrate of Caspase-3. Joint application of Caspase-3 specific inhibitor Z-DQMD-FMK and Rubescensine A together to simultaneously act on Kasumi-1 cells makes apoptosis inhibited, indicating that the activation of Caspase-3 is a key step in inducing cell apoptosis. It is discovered that apoptosis of the cells treated with Rubescensine A is accompanied by reduction of mitochondrial transmembrane potential and activation of Caspase-9, as well as release of mitochondrial cytochrome C. Activation of Caspase-9 is an upstream event corresponding to Caspase-3 activation, and the release of mitochondrial cytochrome C and the activation of Caspase-9 affect the downstream Caspase-3, causing apoptosis, which suggests that Rubescensine A induced Kasumi-1 cell apoptosis is actually the mediated endogenous pathway. In process of Rubescensine A induced Kasumi-1 cell apoptosis of whether there are other apoptotic pathways or mechanisms still needs to be further researched.

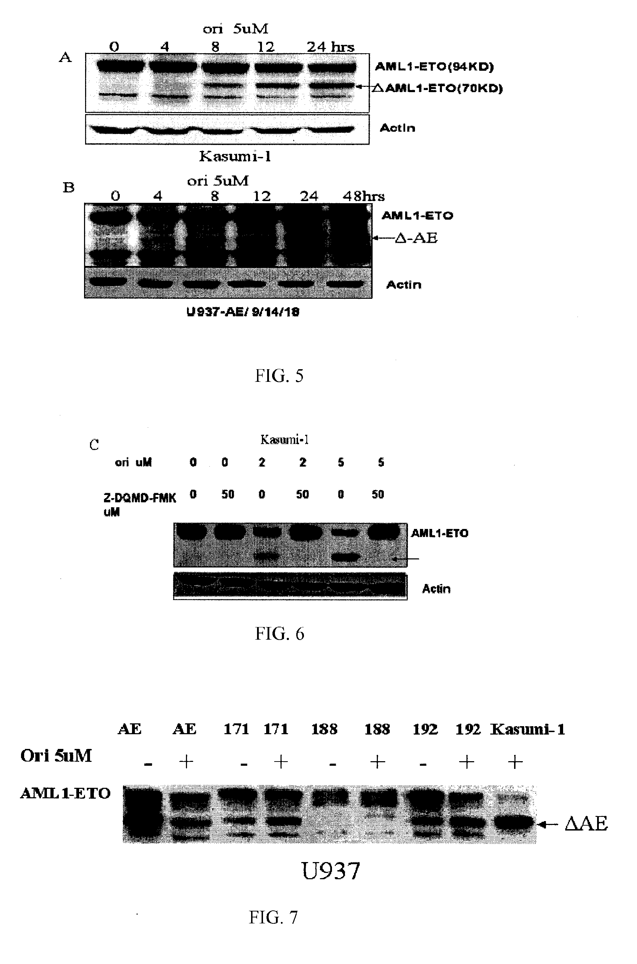

The experiment results show that in the process of Rubescensine A inducing apoptosis of Kasumi-1 cells, degradation of Kasumi-1 cell specific fusion protein AML1-ETO occurs. Application of U937 cell line (U937-A/E) that induces expression of AML1-ETO fusion protein also shows the degradation effect that Rubescensine A induces on AML1-ETO fusion protein (Figure 5).

### Embodiment 3: Study on caspase-3 cut site of AML1-ETO fusion protein

I. Materials and Methods

1. Reagents and equipment: For Rubescensine A and fetal bovine serum, referring to "Materials and Methods" section of Embodiment 1. For Caspase-3 specific inhibitor Z-DQMD-FMK, referring to "Materials and Methods" section of Embodiment 2. Anti-ETO antibody is purchased from American company Santa Cruz Biotech. Actin antibody is purchased from American company Sigma. QuickChange Site-Directed Mutagenesis Kit is a product of American company Stratagene. Plasmid Maxi Preparation Kit is a product of German company QIAGEN. Gene Pulser electroporation instrument and electric transfection cup are products of American company Bio-Rad.

2. Inhibition experiment of Caspase-3 specific inhibitor Z-DQMD-FMK: Kasumi-1 cell cultivation is the same as in Embodiment 1. During the experimental process, cells of density 2-4×10⁵/ml are inoculated and cultured. Incubation is carried out with application of Rubescensine A of two concentrations of 2µM and 5µM with/without 50µM Caspase-3 specific inhibitor Z-DQMD-FMK and then the cells are harvested. The harvested cells are then subjected to total protein western blot inspection.

3. Construction of mutant expression plasmid: QuickChange Site-Directed Mutagenesis Kit is applied for proceeding site-directed mutagenesis for expression plasmid. Plasmid pFLAG-CMV4-AML1-ETO containing full-length AML1-ETO fusion gene is used as a template. Mutation points selected are Asp171, Asp188, Asp192 of AML1-ETO fusion protein to be and the mutations are Asp171Ala, Asp188Ala, Asp192Ala, which are aspartate to alanine mutations.

(1) Primers are designed as shown in the following Table.

**Table 1: Primer applied in site-directed mutagenesis, underlined being base mutations**

| primer | Sequence |
|---|---|
| 171 F | 5'-CAAAATCACAGTGGCCGGGCCCCGAGAACC-3' |
| 171 R | 5'-GGTTCTCGGGGCCCGGCCACTGTGATTTTG-3' |
| 188F | 5'-AGTCCACAATGCCAGCCTCACCTGTGGATG-3' |
| 188R | 5'-CATCCACAGGTGAGGCTGGCATTGTGGAGT-3' |
| 192F | 5'-CCAGACTCACCTGTGGCCGTGAAGACGCAATCT-3' |
| 192R | 5'-AGATTGCGTCTTCACGGCCACAGGTGAGTCTGG-3' |

(2) Mutation chain synthesis reaction: Operations are carried out in accordance with the QuickChange Site-Directed Mutagenesis Kit instructions, and PCR reaction conditions are as follows: 95°C for 1 minute, 95°C for 50 seconds, 60°C for 50 seconds, 68°C for 8 minutes, 68°C for 7 minutes, of which three steps of 95°C for 50 seconds to 68 °C for 8 minutes are for 18 cycles.

(3) Dpnl restriction endonuclease digestion synthetic product. Product of mutant chain synethsis reaction is added with 1µL Dpn I enzyme at 37°C in water bath for 1 hour.

(4) Calcium transformation plasmid, colony selection, plasmid extraction and sequencing, and mass extraction of plasmid.

a. Using XL10-Gold ultracompetent cells of QuickChange Site-Directed Mutagenesis Kit as competent bacteria, taking 45µL of the bacteria, adding 2µLβ mercaptoethanol for mixing then placed on ice for 10 minutes, following by adding 2µL Dpnlenzyme and placing the synthesis product on ice for 30 minutes, then placing in water bath at 42°C for 30 seconds, and on ice for 2 minutes, adding the above-treated competent bacteria to 500µL preheated (at 37°C) LB solution and shaking one hour in the conditions of being at 37°C and for 225 rpm, spreading on board of ampicillin positive LB, incubated in temperature controlled box at 37°C for 16 hours and afterwards observing whether or not there is growth of colonies; and

b. With the observation of the formation of colonies, picking six colonies in each board, multiplying the bacteria, extracting plasmid and preserving the species, selecting plasmid for sequencing thereof. Carrying out mass extraction of the plasmid of which successful mutation is confirmed with sequencing.

4. Transient expression and detection of mutant plasmid: Human leukemia cell line U937 is inoculated, with an initial concentration of 3×10⁵/ml, to RPMI1640 cell culture medium containing 10% FBS and routine culture is carried out under the conditions of 37°C, 5% CO₂, and saturated humidity.

(1) Harvesting U937 cells, centrifuge 200g, for 5-10 minutes;

(2) Using 1640 re-suspending cells containing 0.5% FBS, centrifuge 200g, for 5-10 minutes;

(3) Applying transfection buffer to re-suspend cells to 2×10⁶/ml, positioning 800µl of cells in 1.5ml Eppendorf centrifugation tubes, adding 20µg plasmid and uniformly blending;

(4) Transferring the above-mentioned cell suspension to 0.4cm electric transfection cup (Gene Pulser Cuvette, a product of Bio-Rad company), and carrying out transfection on the Gene Pulser electroporation instrument (product of Bio-Rad company), electric transfection conditions: 280V voltage and 1050µF capacitance;

(5) Placed at room temperature for 10 minutes after the electric transfection; and

(6) Transferring the cells from the electrical transfection cup to 3ml PRMI1640 containing 10% FBS, culturing for 24-48 hours and detecting the expression of mutant plasmid.

5. Effect of Rubescensine A on transient transfection mutant plasmid cells and observing the state of AML1-ETO fusion protein degradation

Transient transfection plasmid containing wild-types or mutant AML1-ETO gene to U937 cells, culturing for 24 hours and afterwards applying 5µM of Rubescensine A to treat the transfection cells. Each mutant is divided into treatment group and control group, and the control group is applied with treatment of the same volume of DMSO and the treatment group is treated with 5µM Rubescensine A. After culture for 24 hours, harvesting cells in routine manner. Total cellular protein is extracted to proceed with Western Blot inspection for detecting degradation of AML1-ETO fusion protein.

II. Results and discussion:

1. Inhibition experiments of Caspase-3 specific inhibitor Z-DQMD-FMK: Rubescensine A induced Kasumi-1 cells apoptosis can be inhibited by Caspase-3 specific inhibitor Z-DQMD-FMK. Western Blot experiments show that Z-DQMD-FMK prevents degradation of AML1-ETO fusion protein (Figure 6).

2. Construction of mutant expression plasmid: Site-directed mutagenesis method is used to obtain three mutant expression plasmids, which provide a basis for subsequent experiments.

3. Treatment of cells transfected with AML1-ETO-mutant plasmid with Rubescensine A and observation of each mutant for AML1-ETO fusion protein degradation: Rubescensine A can lead to apoptosis of cells of transient expression AML1-ETO plasmid. Western Blot experiments show that in U937 cells of transient expression Asp188Ala (D188A) mutant plasmid (Figure 7, 188) with Rubescensine A applied to the cells, no degradation of AML1-ETO fusion protein is found. And in the expression of wild-type (Figure 7, AE), Asp171Ala (Figure 7, 171), and Asp192Ala (Figure 7, 192) mutant cells, Rubescensine A can still lead to degradation of AML1-ETO fusion protein, and the cleaved size of fragment is similar to the cleaved fragment size of AML1-ETO fusion protein in the Kasumi-1 cells.

Hydrolysis sites of Caspases are aspartate (Asp, D). In this experiment, for cells (Kasumi-1, U937-A/E) containing AML1-ETO fusion protein, after being treated with Rubescensine A, the protein shows degradation, and with joint application of Caspase-3 specific inhibitor with Rubescensine A to Kasumi-1 cells, apoptosis and AML1-ETO fusion protein degradation are both prevented, indicating that cleaving of AML1-ETO fusion protein depends on the activation of Caspase-3, so it is inferred that AML1-ETO fusion protein degradation is mediated by Caspase-3. Since degradation of AML1-ETO fusion protein of 94 kDa generates a fragment of 70KDa (the anti-ETO antibodies used in this experiment being anti-ETO protein carboxy-terminal short peptide antibodies), and Caspase-3 normal cut sites comprises the existence of aspartate, the three most likely cutting sites are selected for construction of mutants (Asp171, Asp188, Asp192), mutation being alanine residues (Ala). If the three selected amino acid residues contain Caspase-3 cutting site, treatment with Rubescensine A after mutation can not lead to AML1-ETO fusion protein degradation, and on the contrary, Rubescensine A can still induce degradation of AML1-ETO fusion protein. This experiment shows that expression of AML1-ETO in U937 cells, Asp188Ala mutation can prevent Rubescensine A induced AML1-ETO fusion protein degradation. However, the wild-type and the other two mutations (Asp171Ala, Asp192Ala) can not prevent the AML1-ETO fusion protein degradation. In the expression of wild-type and Asp171Ala, Asp192Ala mutant U937 cells, AML1-ETO fusion protein degradation and the resulting cleaved fragment size is similar to AML1-ETO fusion protein degradation/cleaving manner in Kasumi-1 cells. The above-mentioned results suggest that Asp188 is the key site of Caspase-3 cleaving AML1-ETO fusion protein, while in turn proven that cleaving of AML1-ETO fusion protein is carried out by activated Caspase-3.

### Embodiment 4: Study of therapeutic effect of Rubescensine A on transplant leukemic mice and mechanism thereof

Materials and Methods:

1. Reagents and equipment: For Rubescensine A, propidium iodide, fetal bovine serum, Annexin V Test Kit (ApoAlert Annexin-V kit), referring to "Materials and Methods" section of Embodiment 1. CCK-8 kit is a product of Japanese Institute of Chemistry in Tongren. Flow cytometer, fluorescence microscopy, and cytospin all referring to "Materials and Methods" section of Embodiment 1. Enzyme-linked immunosorbent Detector is a product of American company Bio-Tek.

2. Cytotoxicity experiment with Rubescensine A solubilized by solubilizer: Rubescensine A applied to mouse experiments is a Rubescensine A stock solution that is solubilized by a solubilizer, and solubilization is realized through the selection of a non-ionic solubilizer, pluronic F68, the solubilizer concentration being 1%, solvent being double-distilled water, concentration of Rubescensine A being 2mg/ml, stored at 4°C. Before in vivo mouse experiments are carried out, MTT method is applied for cytotoxicity experiments with the Rubescensine A stock solution that is solubilized by the solubilizer to determine that cytotoxicity of Rubescensine A is not affected by the solubilizer and the solubilizer itself is not cell toxic.

MTT test method is carried out as follows:

(1) Inoculating cells: Kasumi-1 is inoculated with an initial concentration of 2×10⁵/ml, in 10% FBS RPMI1640 cell culture medium and is normally cultured under the conditions of 37°C, 5% CO₂, and saturated humidity to exponential growth phase, and is afterwards adjusted to concentration of 1×10⁵/ml density, each well being of about 1×10⁴ cells inoculated in a 96-well plate, each well having a volume of 90µL.

(2) Treatment with Rubescensine A: Rubescensine A dissolved by DMSO and Rubescensine A solubilized with 1% pluronic F68 are respectively set at three concentrations (0.5µM, 5µM, and 20µM) and are provided with their respective control groups. Each treatment uses three tubes.

(3) Culture of cells: The culture plate is subjected to normal culture for 24 hours under the conditions of 37°C, 5% CO₂, and saturated humidity.

(4) Color: After culture for 24 hours, each well is added with 10µL CCK-8 reagent for normal culture for 1-4 hours under the conditions of 37°C, 5% CO₂, and saturated humidity.

(5) Color comparison: Wavelength of 450-490nm is selected to inspect optical density (OD) of each well with enzyme-linked immunosorbent detector, the results are recorded, and inhibition rate is calculated. Inhibition rate=1-OD_{treatment group}/OD_{control group}.

3. Set-up of nude mouse Kasumi-1 cell subcutaneously-transplanted tumor model and therapeutic effect of Rubescensine A

(1) Kasumi-1 cells are cultured to an exponential growth phase and the cells are counted, number of viable cells being counted, cell density being adjusted to 1.3×10⁸/ml for subsequent use.

(2) For preparation of mice, nude mice of 4-6 weeks old are purchased from the Shanghai Laboratory Animal Center, Chinese Academy of Sciences, and are bred and maintained in a specific pathogen-free environment..

(3) For establishment of model and treatment with Rubescensine A, each of the 4-6 week old mice receives subcutaneous injection of 4×10⁷ Kasumi-1 cells at the right flank, and observation of formation of the transplanted tumor in the mice is made. When the tumor size in the mice can be observed (approximately 100 mm³), three groups are randomly divided, of which the first one is the control group, which is treated with solvent (1% solubilizer Pluronic F68) 0.1ml/10g of body weight via intraperitoneal injection; the second group being applied with treatment of Rubescensine A 7.5mg/kg of body weight via intraperitoneal injection, once per day, for successive application until 12 days after tumor formation; third group being applied with treatment of Rubescensine A 15mg/kg of body weight via intraperitoneal injection, once per day, for successive application until 12 days after tumor formation. The growth of the transplanted tumors and the tumor sizes are observed among the three groups. Upon completion of the treatment, the mice are killed for pathomorphological inspection of the transplanted tumors, and pathomorphological inspection of important organs, such as spleen, liver, bone marrow, and kidneys, is also made to identify organizational abnormal cell infiltration for each group.

4. Set-up of AEtr transplant mouse model and therapeutic effects of Rubescensine A

(1) To prepare spleen cells of AEtr transplant mice, retrovirus is used to transfect AML1-ETO fusion gene with C-terminal deletion of 200 amino acids into bone marrow mononuclear cells of C57 mice, and these cells are used as donor cells. At the same time, C57 mice are subjected to 10 cGy of radiation exposure (lethal dose radiation) to serve as recipient mice. The donor cells are transplanted into the recipient mice, and the mice are raised in specific pathogen free (SPF) environment. After leukemia cells occur in the mice, spleen cells are taken for use in the subsequent experiments.

(2) Preparation of mice: C57 mice of 6-8 weeks old are purchased from the Shanghai Laboratory Animal Center, Chinese Academy of Sciences, and are bred and maintained in a specific pathogen-free environment..

(3) Spleen cell transplantation and subculture of AEtr transgenic mouse morbidity: The 6-8 week-old C57 mice are subjected to exposure to 400cGy radiation, and in 24 hours, each mouse receives injection of 1×10⁶-2×10⁶ spleen cells of AEtr transplant mouse (cells obtained from the above-mentioned step 1). Activity of the mice is observed, and after two weeks, routine blood test is started to carry out every week and morphology of peripheral blood cell is assessed. When lots of leukemic cells are observed in peripheral blood, the mice are sacrificed via cervical dislocation method, and the bone marrow cells and spleen cells are taken and smeared with Cytopro centrifuge and dried, followed by Wright staining. Cell morphology is observed with microscopes. Some of the spleen cells are used for subculture. The remaining bone marrow cells and spleen cells are cryo-preserved in liquid nitrogen for subsequent uses. To set up the second generation of transplanted mice, each mouse receives injection of 3×10⁶ cells via tail vein.

(4) Mice bearing AEtr-expressing leukemic cells are set up and randomly divided into 6 groups, of which the first one is the control group, which is subjected to application of solvent (1% solubilizer Pluronic F68) 0.1ml/10g of body weight by intraperitoneal injection; the second group being applied with low-dose cytarabine (Ara-CL), the third group being treated with high-dose cytarabine (Ara-CH), the fourth group receiving treatment of Rubescensine A 2.5mg/kg of body weight via intraperitoneal injection, once per day for consecutive five days, then every 2 days for consecutive two weeks, the fifth group receiving treatment of Rubescensine A 7.5mg/kg of body weight via intraperitoneal injection, once per day for consecutive five days, then every 2 days for consecutive two weeks, and the sixth group receiving treatment of Rubescensine A 15mg/kg of body weight via intraperitoneal injection, once per day for consecutive five days, then every 2 days for consecutive two weeks. The treatment is carried out at the 7th day after transplantation. During the experiment period, observation of general condition, survival period, body weight, and peripheral blood is made. When the mice become moribund, the mice are killed to remove the spleen for observing the size, spleen and bone marrow cells are collected, and pathomorphological inspection of important organs, such as spleen, liver, bone marrow, and kidneys, is carried out to identify organizational abnormal cell infiltration.

Results and discussion:

1. Cytotoxicity of 1% F68 (pluronic) solubilized Rubescensine A (2mg/ml) on Kasumi-1 is the same as that of DMSO dissolved Rubescensine A, and solvent of 1% pluronic F68 is not toxic to Kasumi-1 cells (results not shown).

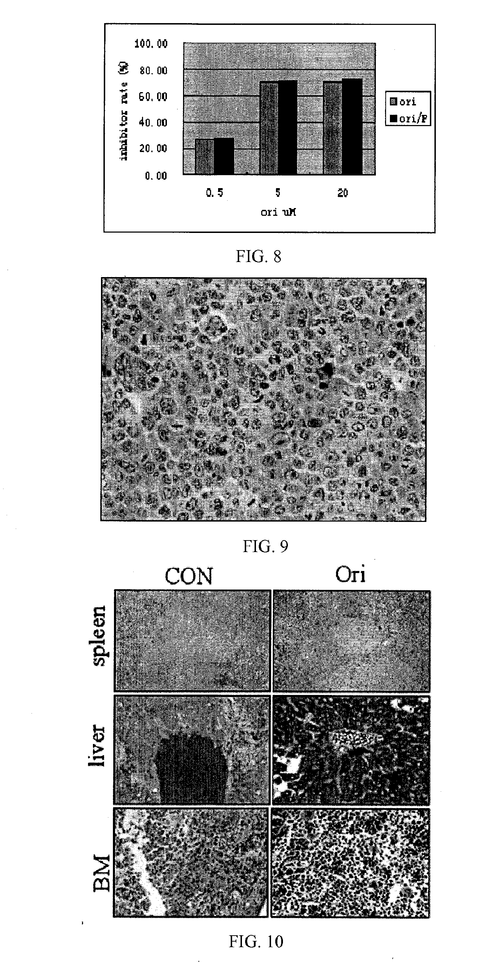

2. Set-up of nude mouse Kasumi-1 cell subcutaneously transplanted tumor model: The nude mouse Kasumi-1 cell subcutaneously transplanted tumor model is set up by using standard process of cell line transplanted subcutaneously in nude mice. Kasumi-1 cells are cultured to exponential growth phase to adjust to cell density of 1.3×10⁸/ml. 4-6 week-old nude mice are used and each mouse receives subcutaneous injection of 4×10⁷ Kasumi-1 cells at the right flank. Formation of transplanted tumor in the mice is observed and tumor volume is calculated, in which tumor volume = length × width² × π/6. When the tumor size in the mice can be observed (at this time, the volume being about 100 mm³), the mice are randomly divided into groups. The formation of the tumor in the nude mice is 5-16 days after the transplantation, and all animals (100%) develop a measurable tumor. In morphological examination and pathological examination of important organs, a large number of Kasumi-1 cells are observed in the tumor of the mice (Figure 9), and these cells can also be seen in spleen, liver, and bone marrow, particularly in the spleen which is filled with a large number of abnormal cells, leading to the loss of normal structure in spleen (Figure 10, CON).

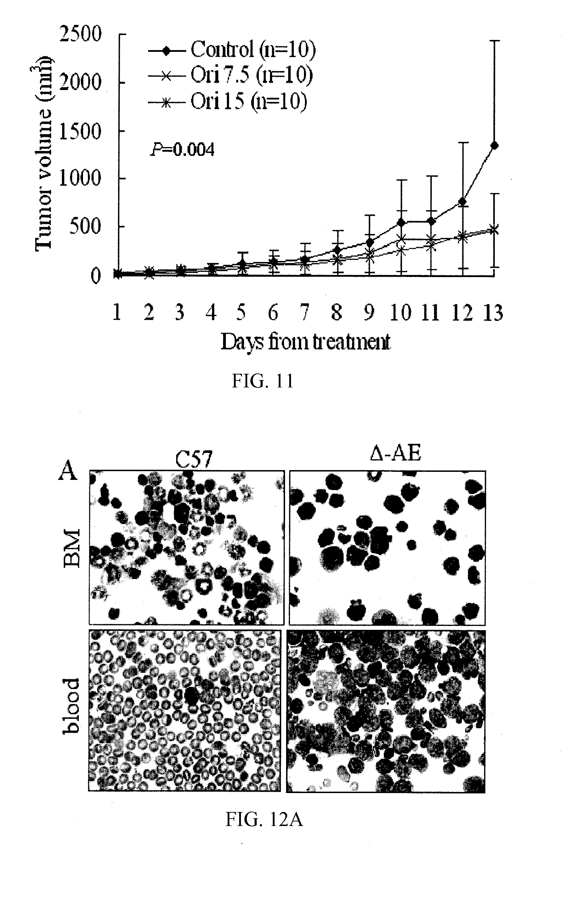

3. Rubescensine A can inhibit the growth of nude mouse Kasumi-1 cell subcutaneously transplanted tumor: At the time when tumors growth in the mice can be observed, random division is made for three groups, of which the first one is the control group, that receives solvent (1% solubilizer Pluronic F68) 0.1ml/10g of body weight via intraperitoneal injection, the second group being applied with treatment of Rubescensine A 7.5 mg/kg of body weight via intraperitoneal injection, once per day, continued until the 12th day of tumor formation, the third group being applied with treatment of Rubescensine A 15mg/kg of body weight via intraperitoneal injection once per day, application continued until the 12th day of tumor formation. It is discovered by pathological detection that in Rubescensine A treatment groups, abnormal cells in spleen is significantly less than the control group, and abnormal tumor cells in liver and bone marrow are rarely seen (Figure 10, Ori). Observation of the three groups of transplanted tumor growth shows that transplanted tumor growth is slower in the mice of the treatment groups than in the control group (Figure 11). The control group and the Rubescensine A 7.5mg/kg group have significant difference (P=0.002, n=10); the control group and the Rubescensine A 15mg/kg group have significant difference (P=0.001, n=10); and there is no significant difference between the Rubescensine A 7.5mg/kg group and the Rubescensine A 15mg/kg group (P=0.892).

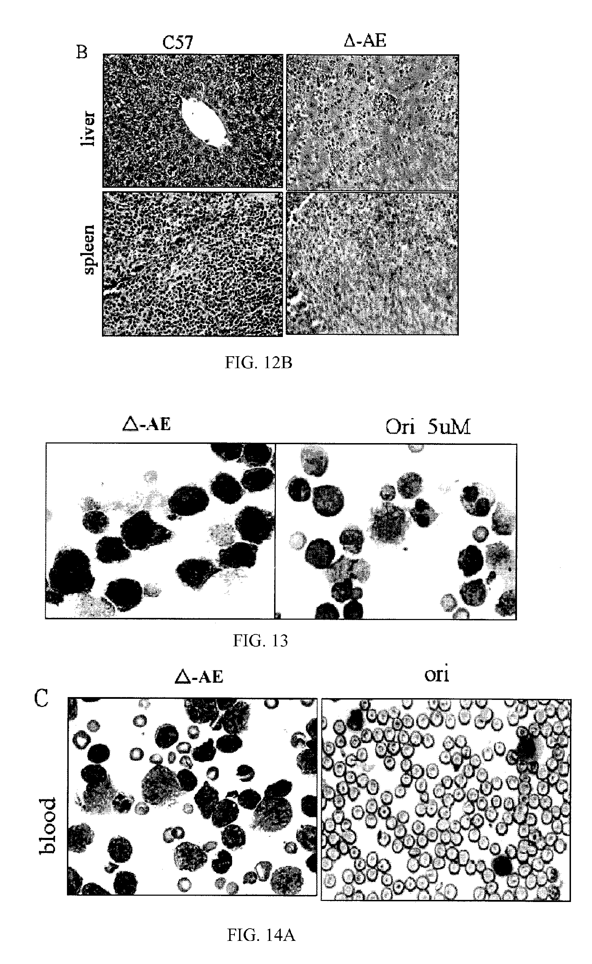

4. Set-up of AEtr transplant mouse model: Compared with normal C57 mice, peripheral blood and bone marrow of AEtr transplant mice are filled with large numbers of immature cells (Figure 12A), these cells being bulky in size and having a high proportion of nucleoplasm, nucleus being mostly round or oval shaped. In addition, the spleen of the ill mice is significantly enlarged. Spleen infiltration is very serious, showing a large number of leukemia cells, and normal spleen nodule structure is damaged. In the neighborhood of liver blood vessels as well as the liver blood sinus, it can also be observed the existence of substantial infiltration of leukemia cells (Figure 12B, C57).

5. Rubescensine A can induce in vitro spleen cells apoptosis of AEtr transplant mice: Leukemia cells separated from spleen of the AEtr transplanted mice are cultured in vitro in 10% FBS in RPMI1640 cell culture medium, and Rubescensine A 5µM is added for the observation of the effect of apoptosis induced thereby. After 24 hour treatment with Rubescensine A, Annexin V/PI double-staining shows that 57.4% of cell apoptosis has occurred, but for corresponding cells that are in vitro cultured for 24 hours and do not receive treatment of Rubescensine A, 36.3% of cell apoptosis occurs. Annexin V/PI double-staining analysis indicates that Rubescensine A treatment group has a significantly increased apoptotic cells proportion, although the spleen cells in vitro cultured control group also has apoptosis. Meanwhile, the morphology shows that in vitro treatment of Rubescensine A for 24 hours shows a large number of apoptotic cells, with characteristics of early apoptosis, such as breakdown of karyopyknosis; and some cells show secondary necrosis (Figure 13).

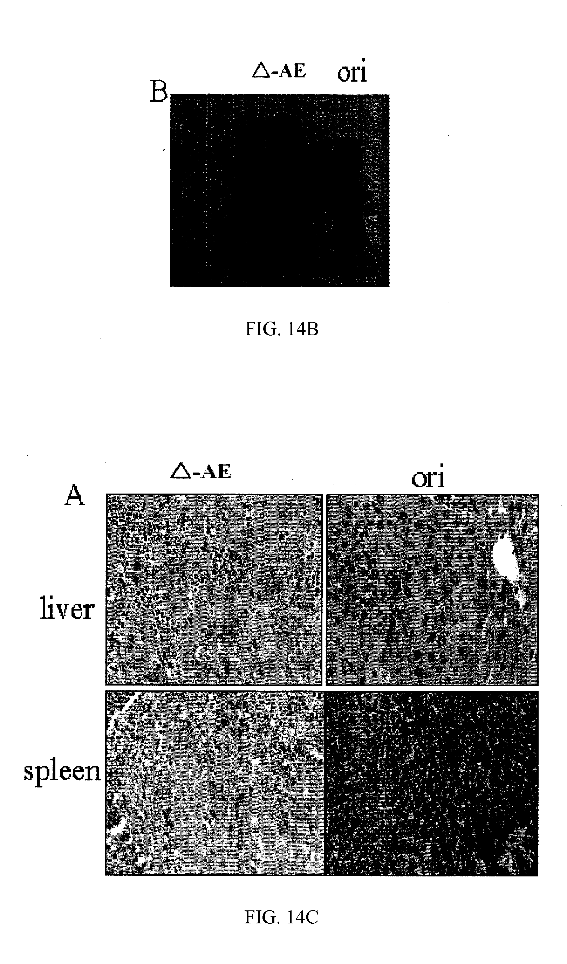

6. Rubescensine A can extend survival period of AEtr transplant mice, and reduce infiltration of leukemic cells. The AEtr transplant mice are randomly divided into 6 groups, treatment is applied to the mice as described in "Materials and Methods" section. Peripheral blood smear shows that AEtr transgenic transplanted mice have a large number of leukemic cells, which show morphologically a large cell size, a high proportion of nucleoplasm, nucleus being not lobulated and mostly in circular shape, while the Rubescensine A treatment group has less of the above-mentioned cells (Figure 14A). When the mice become moribund, the mice are killed via cervical dislocation method and dissection of the mice of the control group and the drug treatment group is done for pathological inspection. It is discovered that the mice of the treatment group have significantly smaller spleen than the disease mouse group (Figure 14B). Pathological inspection of liver and spleen shows that the liver and spleen of the control group contain a large number of leukemia cells, but the mice that are treated with Rubescensine A show significantly reduced infiltration in liver and spleen, leukemic cells are significantly less, and liver and spleen can be restored to the normal structure (Figure 14C).

Comparison of mouse survival period: Mice in the control group begin to die at the 22nd day, and all die at the 27th day, the average survival period being 23.8 days, the median survival period of 23.0 days; mice in the Rubescensine A 2.5mg/kg/day group begin to die at the 25th day, and all die at the 31st day, the average survival period being 28.3 days, the median survival period 28.0 days; mice in the Rubescensine A 7.5mg/kg/day group begin to die at the 24th day, and all die at the 36th day, the average survival period being 29.8 days, the median survival period 30 days; and mice in the Rubescensine A 15mg/kg/day group begin to die at the 24th day, and all die at the 36th day, the average survival period being 31.3 days, the median survival period 32 days. Different treatment groups in survival period show statistically significant difference (P=0.0001), indicating that Rubescensine A can significantly prolong the survival period of the AEtr transplant mice and therefore has a definite and excellent therapeutic effect on leukemia. The efficacies of Rubescensine A and cytarabine on AEtr transplant leukemia mouse model are also compared, and it is discovered that Rubescensine A at 2.5mg/kg/day for 10 days produces similar efficacy as treatment with cytarabine 25 mg/kg/day for 5 days (low-dose cytarabine), and 7.5, 15mg/kg/day Rubescensine A has better therapeutic effect than small dose cytarabine; the efficacy of treatment of 25mg/kg/day cytarabine for 10 days (high-dose cytarabine) in some leukemic mice (4/9) is better in comparison with Rubescensine A, but leads to early death (survival period of only 18 days) for the other mice (5/9). Different from high-dose cytarabine, dosage of 2.5-15mg/kg/day Rubescensine A does not cause bone marrow suppression or weight reduction in mice. Thus, in the treatment of leukemia, Rubescensine A has a definite therapeutic effect and characteristics of minor drug toxic side effects.

Poloxamer188 (Pluronic F68), which is a polyethylene-polypropylene block copolymer, is one of a handful of synthetic emulsifier currently used for intravenous emulsion, and is also a non-ionic solubilizer. Pluronic F68 is utilized in the present experiments to increase the solubility of Rubescensine A.

Through the present invention, it is discovered that Rubescensine A of Isodon diterpenoids can extend the survival period of the AEtr transplant mice, and the compound can also inhibit the growth of Kasumi-1 cells of subcutaneously transplanted tumor in nude mice , and at the same time Rubescensine A can reduce the infiltration of tumor cells in mice of these two models; leukemia cells in vitro culture experiments show that Rubescensine A can rapidly induce in vitro leukemia cell apoptosis of the AEtr transplant mice. The above-mentioned results show that the compound has strong anti-leukemia efficacy.

At present, chemotherapy is the main treatment for leukemia, supplemented by transplantation of bone marrow and umbilical cord blood. All-trans retinoic acid (ATRA) differentiation therapy, As₂O₃ apoptotic therapy and joint application of the two have made people move forward on this special type of APL leukemia treatment with remarkable landmark. As for the other types of acute leukemia, chemotherapy is still the main treatment. Therefore, the overwhelming majority of researchers have urgent missions of continuing with the development of novel, safe, and effective anti-leukemia drug.

To summarize the above-mentioned comprehensive studies, treatment effect of Rubescensine A in AEtr transplantation mouse model and the nude mouse Kasumi-1 cell subcutaneously transplanted tumor model has shown good prospect of the compound's clinical application, with the potential to become a new anti-leukemia drug.

### Embodiment 5: Study of effect of Rubescensine A on other leukemic cells

I. Materials and Methods:

1. Reagents and equipment: Rubescensine A with a purity of 98%-99.8% is subjected to dissolution by application of DMSO (Sigma) for preparing a stock solution of 10⁻²mol/L concentration stored at -20°C. Propidium iodide (PI), which is a product of American company Sigma, is mixed with triple-distilled water to form 250µg/mL stock solution stored at 4°C and avoiding light. Fetal bovine serum is purchased from American company HyClone. Annexin V Test Kit (ApoAlert Annexin-V kit) is purchased from American company BD Biosciences. Flow cytometer is a product of American company Beckman Coulter. Fluorescence microscope (Olympus, BX60) and phase contrast microscope (Olympus, IMT) are purchased from Japanese corporation Olympus. Smear instrument cytospin is purchased from British company Shandon.

2. Cell culture and treatment and morphological observation of cells: AML M2 cell line HL-60 without t(8;21) chromosomal translocation, acute monocytic leukemia U937 cell line, chronic myelogenous leukemia cell line K562, acute promyelocytic leukemia cell line NB4, and primary leukemia cells from patients of AML M2 without t(8;21) chromosomal translocation and patients of acute promyelocytic leukemia and chronic myelogenous leukemia.

The above-mentioned cell lines are incubated, with an initial concentration of 2×10⁵/ml, in RPMI1640 cell culture medium containing 10% FBS and are normally cultured under the conditions of 37°C, 5% CO₂, and saturated humidity. In the exponential growth period, the cell density is adjusted to 3×10⁵/ml, and is applied with treatment of Rubescensine A, in which specific application of three concentrations, 8µM, 20µM, and 25µM, is given and in 5µm concentration, time points of 0h, 4h, 8h, 12h, and 24h are selected. The control group is applied with the greatest volume of DMSO that is added to the treatment groups. After the treatment is completed, cells are inspected with the flow cytometer. The cells undergo apoptosis, and Cytopro centrifuge is applied to smear and then drying is carried out, followed by Wright staining, and afterwards, cell morphology is observed with the microscope.

3. Apoptosis inspection with Annexin V method: Operations are carried out in accordance with the Annexin V Test Kit specification, with the following steps:

(1) Harvesting respectively (5-10)×10⁵ HL-60, U937, K562, NB4 cells, where the cells are washed two times with pre-cooled 1×PBS and afterwards washed one time with Binding Buffer from the Test Kit and re-suspended in 100µL Binding Buffer;

(2) Adding 5µL Annexin V-FITC reagent and 5µL PI, gently mixing, and incubating at room temperature for 15-20 minutes in a condition of avoiding light; and

(3) Adding 400µL Binding Buffer, and carrying out inspection with flow cytometer in one hour.

II. Results and discussion:

Rubescensine A can induce occurrence of apoptosis of HL-60, U937, K562, NB4 cell, and this effect is dose-and time-dependent. The Rubescensine A treated cells show morphologically significant apoptotic features, such as chromatin condensation, nuclear breakdown and cell membrane maintaining integrity. In the apoptotic process, the imbalance distribution of phospholipids in bimolecular layer of cell membrane is destroyed, and eversion of phosphatidylserine (PS) from inner layer to outer layer occurs, whereby the phosphatidylserine exposed on the outer surface may be recognized and engulfed by nearby phagocytes, so that apoptotic cells can be cleared and removed. Annexin V is a phospholipid-binding protein, which can specifically identify the everted phosphatidylserine on the cell surface, and so it is often used for detection of apoptosis. PI is a red fluorescent dye that can combine DNA, but only in cells undergoing necrosis, under cell membrane rupture circumstances, it can go through the cell membrane to bind with DNA. Therefore, flow cytometry analysis for double-staining of Annexin V-FITC/PI is the most sensitive and specific method for detecting apoptosis suspending cells. The results of Annexin V-FITC/PI double-staining show that Annexin V(+)/PI(-) cells and the effect of Rubescensine A are closely related, but showing time-dependency. After treatment for 4 hours, Annexin V(+)/PI(-) cells appear. Subsequently, Annexin V (+)/PI(+) cells increase their number with prolongation of time.

Although Rubescensine A can induce the occurrence of apoptosis of these cells, it needs a higher concentration of 8-25µM.

### Embodiment 6: Experiments of joint application of Rubescensine A and cytarabine significantly improving therapeutic effect on M2-type acute myeloid leukemia and prolonging survival period of experiment mice

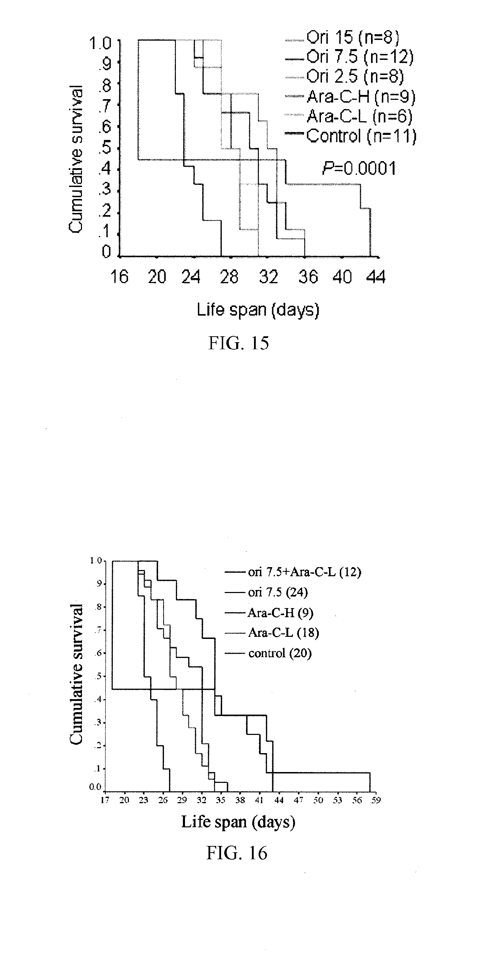

Rubescensine A and cytarabine applied in combination can significantly improve the effect of both of them on the M2-type acute myeloid leukemia and prolong the survival period of experiment mice, see Figure 16.

Set up leukemia mouse model With AEtr gene, and randomly divide into 6 groups, of which the first is the control group, applying solvent (1% solvent Pluronic F68) 0.1ml/10g of body weight to carry out intraperitoneal injection; second group is applied with low-dose cytarabine (Ara-C-L, 25mg/kg/day consecutive for 5 days), the third group is applied with treatment of high-dose cytarabine (Ara-C-H, 25mg/kg/day consecutive for 10 days), the fourth group is applied with treatment of Rubescensine A 7.5mg/kg/day via intraperitoneal injection, the fifth group is applied with treatment of Rubescensine A 7.5mg/kg/day as well as low-dose cytarabine, once per day, consecutively for 5 days, then every 2 days consecutively for two weeks. Treatment is carried out at the 7th day after transplantation. During the experiment period, observation of the general condition, survival period, body weight, and peripheral blood of the mice is made. When the mice become moribund, the mice are killed to remove the spleen for observing the size, spleen and bone marrow cells are collected, and pathomorphological inspection of important organs, such as spleen, liver, bone marrow, and kidneys, is carried out to identify organizational abnormal cell infiltration. For AML M2 leukemia mice, treatment with a combination of Rubescensine A and cytarabine can further prolong the survival period of mice so that the median survival period of the mice can be extended to 34.1 days (compared with cytarabine alone group, P=0.0006; and compared with Rubescensine A 7.5mg/kg/day alone group, P=0.0014).

### Embodiment 7: Experiments of joint application of Rubescensine A and cytarabine reducing leukemia cells infiltration in peripheral blood, liver, and spleen

Rubescensine A and cytarabine applied in combination can reduce leukemic cells infiltration in peripheral blood, liver, and spleen, see Figure 17.

Set up leukemia mouse model with AEtr gene, and randomly divide into 6 groups, of which the first group is the control group, applying solvent (1% solvent Pluronic F68) 0.1ml/10g of body weight via intraperitoneal injection; the second group is applied with low-dose cytarabine (Ara-C-L, 25mg/kg/day consecutive for 5 days), the third group is applied with treatment of high-dose cytarabine (Ara-C-H, 25mg/kg/day consecutive for 10 days), the fourth group is applied with treatment of Rubescensine A 7.5mg/kg/day via intraperitoneal injection, the fifth group is applied with treatment of Rubescensine A 7.5mg/kg/day as well as low-dose cytarabine, once per day, consecutively for 5 days, then every 2 days consecutively for 2 weeks. Treatment is carried out at the 7th day after transplantation. During the experiment, observation of the general condition of mice, survival period, body weight, peripheral blood, and so on is made. When the mice become moribund, the mice are killed to remove the spleen for observing the size, spleen and bone marrow cells are collected, pathomorphological inspection of important organs, such as spleen, liver, bone marrow, and kidneys, is carried out to identify organizational abnormal cell infiltration. For AML M2 leukemia mice, treatment with a combination of Rubescensine A and cytarabine can significantly reduce the percentage of leukemic cells in peripheral blood of the leukemic mice, reduce leukemic cell infiltration in liver, and reduce leukemic cell infiltration in spleen, making the enlarged spleen substantially restoring to normal.

### Embodiment 8: Experiments of sequential and joint application of Rubescensine A (7.5mg/kg/day IP) with G-CSF (50mg/kg/day, SC)+ATRA (10mg/kg/day IP) improving therapeutic efficacy on M2-type acute myeloid leukemia and prolonging survival period of experiment mice

Rubescensine A (7.5mg/kg/day, IP) and G-CSF (50mg/kg/day, SC)+ATRA (10mg/kg/day, IP) sequentially and jointly applied can significantly improve the therapeutic effect of both of them on M2-type acute myeloid leukemia and prolongs the survival period of experiment mice, see Figure 18.

Set up leukemia mouse model with AEtr gene, and randomly divide into 3 groups, of which the first is the control group (n=9), which is subjected to injection with control solvent DMSO 0.2ml/10g via intraperitoneal injection; the second group: G-CSF+ATRA→ori Group: G-CSF (50mg/kg/day, SC)+ATRA (10mg/kg/day IP) for 3 days→Rubescensine A for 3 days→G-CSF (50mg/kg/day SC)+ATRA (10mg/kg/day IP) for 4 days→Rubescensine A for 4 days (n=7); ori→G-CSF+ATRA group: Rubescensine A for 3 days→G-CSF (50mg/kg/day)+ATRA (10mg/kg/day) for 3 days→Rubescensine A for 4 days→G-CSF (50mg/kg/day)+ATRA (10mg/kg/day) for 4 days (n=8). During the experiment, observation of the general condition of mice, survival period, body weight, peripheral blood, and so on is made. Two hours after treatment administration, the mice are killed to remove the spleen for observing the size, spleen and bone marrow cells are collected, and pathomorphological inspection of important organs, such as spleen, liver, bone marrow, and kidneys, is carried out to identify organizational abnormal cell infiltration. For AML M2 leukemia mice, sequential and joint application of Rubescensine A (7.5mg/kg/day IP) and G-CSF (50mg/kg/day, SC)+ATRA (10mg/kg/day IP) can significantly reduce the percentage of leukemic cells in peripheral blood and bone marrow, and reduce leukemic cell infiltration in liver. And, the survival period of experiment mice is prolonged.

### Embodiment 9: Experiments of therapeutic effect of Rubescensine A amino acid derivatives on M2-type acute myeloid leukemia and prolonging survival period of experiment mice

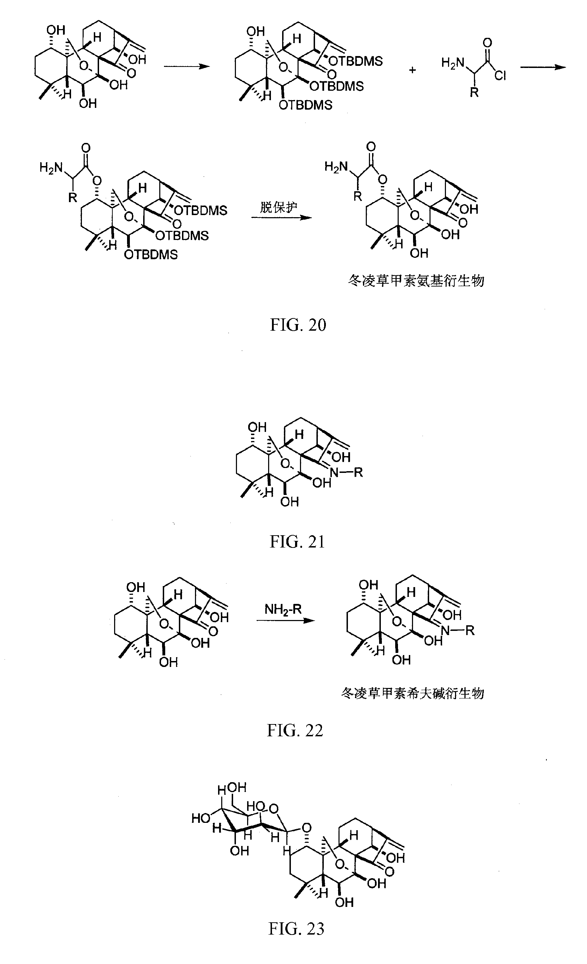

Rubescensine A amino acid derivatives (hydroxy reacting with amino acids to generate derivatives containing amino acids) has a chemical structural formula shown in Figure 19. Method for synthesizing Rubescensine A amino acid derivatives is illustrated in the flow chart (see Figure 20).

Kasumi-1 cells are cultured in accordance with the method described in Embodiment 1, and 0.5-15µM Rubescensine A amino acid derivatives are used to treat Kasumi-1 cells. Afterwards, detection of growth inhibition and apoptosis induction of Kasumi-1 cells is made in accordance with the method described in Embodiment 1. It is discovered that the derivatives are also effective in inhibiting growth of Kasumi-1 cells and promoting apoptosis of Kasumi-1 cells, showing definite anti-leukemia effect.

### Embodiment 10: Experiments of therapeutic effect of Rubescensine A Schiff base derivatives on M2-type acute myeloid leukemia and prolonging the survival period of experiment mice

Rubescensine A Schiff base derivatives (carbonyl of Rubescensine A chemical structure reacting with amines to generate Schiff base) has a chemical structural formula shown in Figure 21. Method for synthesizing Rubescensine A Schiff base derivatives is illustrated in the flow chart (see Figure 22).

Using the above-mentioned method, it can obtain that the derivatives are also effective in inhibiting the growth of Kasumi-1 cells and promoting apoptosis of Kasumi-1 cells, showing definite anti-leukemia effect.

### Embodiment 11: Experiments of therapeutic effect of Rubescensine A glycosyl derivatives on M2-type acute myeloid leukemia and prolonging survival period of experiment mice

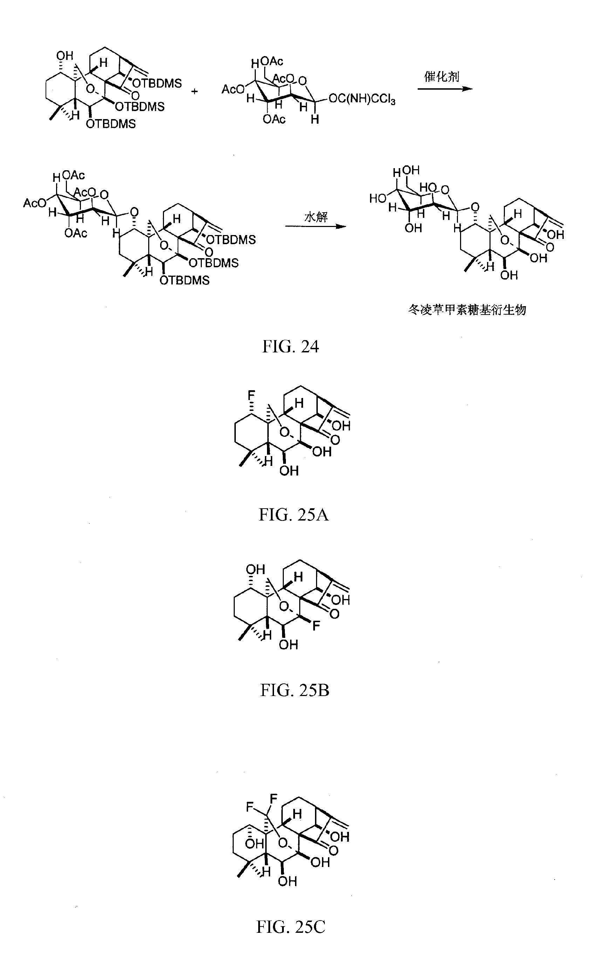

Rubescensine A glycosyl derivative (hydroxy reacting with sugar to generate sugar derivatives) has a chemical structural formula shown in Figure 23. Method for synthesizing Rubescensine A glycosyl derivatives is illustrated in the flow chart (see Figure 24).

Using the above-mentioned method, it can obtain that the derivatives are also effective in inhibiting the growth of Kasumi-1 cells and promoting apoptosis of Kasumi-1 cells, showing definite anti-leukemia effect.

### Embodiment 12: Experiments of therapeutic effect of Rubescensine A fluorine derivatives on M2-type acute myeloid leukemia and prolonging survival period of experiment mice

Rubescensine A fluorine derivatives (hydroxyl and methylene site connecting fluoride (F) to form fluorine-containing derivatives) have chemical structural formula shown in Figure 25.

Using the above-mentioned method, it can obtain that the derivatives are also effective in inhibiting the growth of Kasumi-1 cells and promoting apoptosis of Kasumi-1 cells, showing definite anti-leukemia effect.

## Claims

1. Application of Rubescensine A in manufacturing drugs for treating acute leukemia.

2. The application according to Claim **1,** wherein Rubescensine A was applied in manufacturing drugs for treating acute myeloid leukemia with AML1-ETO fusion gene.

3. The application according to Claim **2,** wherein leukemia cells are of a concentration of 1-20x10⁵cells/ml, and amount of Rubescensine A used is 0.5-5µM.

4. The application according to Claim **3,** wherein leukemia cells are of a concentration of 1-20x10⁵cells/ml, and amount of Rubescensine A used is 2-5µM.

5. The application according to Claim **1,** wherein Rubescensine A is used in manufacturing a drug for treating acute myeloid leukemia containing no AML1-ETO fusion gene.

6. The application according to Claim **1,** wherein Rubescensine A is used in manufacturing a drug for treating acute monocytic leukemia.

7. The application according to Claim **1,** wherein Rubescensine A is used in manufacturing a drug for treating acute promyelocytic leukemia.

8. The application according to Claim 5, 6, or 7, wherein leukemia cells are of a concentration of 1-20 x10⁵cells/ml, and amount of Rubescensine A used is 8-25µM.

9. Application of Rubescensine A in manufacturing a drug for treating chronic myelogenous leukemia.

10. The application according to Claim **9,** wherein leukemia cells are of a concentration of 1-20x10⁵cells/ml, and amount of Rubescensine A used is 8-25µM.

11. Application of Rubescensine A jointly with cytosine arabinoside in manufacturing a drug for treating M2-type acute myeloid leukemia.

12. The application according to Claim **11,** wherein amount of Rubescensine A used is 7.5mg/kg/day and cytosine arabinoside is 25mg/kg/day, consecutively for 5 days.

13. Application of Rubescensine A sequentially applied in combination with G-CSF + ATRA in manufacturing a drug for treating M2-type acute myeloid leukemia.

14. The application according to Claim **13,** wherein Rubescensine A (7.5mg/kg/day IP) and G-CSF (50mg/kg/day, SC) + ATRA (10mg/kg/day IP) are sequentially applied in combination.

15. Application of Rubescensine A derivatives in manufacturing a drug for treating acute leukemia.

16. The application according to Claim **15,** wherein the Rubescensine A derivatives are Rubescensine A Schiff base derivatives.

17. The application according to Claim **15,** wherein the Rubescensine A derivatives are Rubescensine A amino derivatives.

18. The application according to Claim **15,** wherein the Rubescensine A derivatives are Rubescensine A glycosyl derivatives.

19. The application according to Claim **15,** wherein the Rubescensine A derivatives are fluorine-containing derivatives.

20. Application of Rubescensine A in manufacturing a drug for treating esophageal cancer.

21. Application of Rubescensine A in manufacturing a drug for treating gastric cancer.

22. Application of Rubescensine A in manufacturing a drug for treating colon cancer.

23. Application of Rubescensine A in manufacturing a drug for treating liver cancer.

24. Application of Rubescensine A in manufacturing a drug for treating lung cancer.

25. Application of Rubescensine A in manufacturing a drug for treating pancreatic cancer.

26. Application of Rubescensine A in manufacturing a drug for treating prostate cancer.

27. Application of Rubescensine A in manufacturing a drug for treating melanoma.

28. Application of Rubescensine A in manufacturing a drug for treating nasopharyngeal carcinoma.
